# EUROPÄISCHE PATENTSCHRIFT

(11) **EP 3 946 240 B1**
(45) Veröffentlichungstag und Bekanntmachung des Hinweises auf die Patenterteilung: **15.10.2025**
(21) Anmeldenummer: 20704474.4
(22) Anmeldetag: 05.02.2020
(51) Int. Cl.: A61K 8/58, A61K 8/19, A61K 8/34, A61K 8/368, A61K 8/37, A61Q 5/06

(54) **ERHÖHUNG DER STABILITÄT VON MITTELN ZUR BEHANDLUNG VON KERATINMATERIAL**
INCREASING THE STABILITY OF AGENTS FOR TREATING KERATIN MATERIAL
RENFORCEMENT DE LA STABILITÉ D'AGENTS UTILISÉS POUR TRAITER DE LA MATIÈRE KÉRATINIQUE

(30) Priorität: 04.04.2019 DE 102019204804
(43) Veröffentlichungstag der Anmeldung: 09.02.2022
(73) Patentinhaber: Henkel AG & Co. KGaA, 40589 Düsseldorf (DE)
(72) Erfinder: LECHNER, Torsten, 40764 Langenfeld (DE); WESER, Gabriele, 41472 Neuss (DE); KOLONKO, Claudia, 42857 Remscheid (DE); KRIENER, Caroline, 40229 Düsseldorf (DE); SCHUMACHER, Ulrike, 40589 Düsseldorf (DE); NOWOTTNY, Marc, 41069 Mönchengladbach (DE); SCHOEPGENS, Juergen, 41366 Schwalmtal (DE); JAISER, Phillip, 40764 Langenfeld (DE); MATHIASZYK, Carsten, 45277 Essen (DE)
(86) Internationale Anmeldenummer: PCT/EP2020/052810
(87) Internationale Veröffentlichungsnummer: WO 2020/200545

(56) Entgegenhaltungen:
- DE-A1- 10 200 185
- DE-A1- 102011 089 060
- US-A1- 2006 112 500
- US-A1- 2016 235 657
- NN: "Schwarzkopf Professional Igora Royal", 5 May 2020 (2020-05-05), XP055691679, Retrieved from the Internet <URL:https://www.hair-express.de/Schwarzkopf-Igora-Royal-60-ml> [retrieved on 20200505]

## Beschreibung

Die vorliegende Anmeldung liegt auf dem Gebiet der Kosmetik und betrifft ein Verfahren zur Behandlung von keratinischem Material, insbesondere menschlichen Haaren, welches die Anwendung von zwei Zusammsetzungen (A) und (B) umfasst, die unmittelbar vor der Anwendung miteinander vermischt werden. Bei der Zusammensetzung (A) handelt es sich um eine wasserarme Zubereitung, die mindestens zwei organische C₁-C₆-Alkoxysilane enthält, und die Zusammensetzung (B) beinhaltet Wasser sowie mindestens eine spezielle aromatische Verbindung einer bestimmten Formel.

Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus.

EP 2168633 B1 beschäftigt sich mit der Aufgabenstellung, langanhaltende Haarfärbungen unter Einsatz von Pigmenten zu erzeugen. Die Schrift lehrt, dass sich bei Verwendung einer Kombination aus Pigment, organischer Silicium-Verbindung, hydrophobem Polymer und einem Lösungsmittel auf Haaren Färbungen erzeugen lassen, die gegenüber Schamponierungen besonders widerstandsfähig sind.

Bei den in der EP 2168633 B1 eingesetzten organischen Silicium-Verbindungen handelt es sich um reaktive Verbindungen aus der Klasse der Alkoxy-Silane. Diese Alkoxy-Silane hydrolysieren in Gegenwart von Wasser mit hoher Geschwindigkeit und bilden - abhängig von den jeweils eingesetzten Mengen an Alkoxy-Silan und Wasser - Hydrolyseprodukte und/oder Kondensationsprodukte aus. Der Einfluss der in dieser Reaktion eingesetzten Wassermenge auf die Eigenschaften des Hydrolyse- bzw. Kondensationsproduktes werden beispielsweise in WO 2013068979 A2 beschrieben.

DE 102011089060 A1 betrifft die Verwendung einer Wirkstoffkombination (a) und (b) zur Verbesserung künstlicher Färbungen farbgebender Verbindungen auf keratinischen Fasern. Bei (a) handelt es sich um ein wasserlösliches Polymer, und (b) stellt eine polare Alkoxysilanverbindung dar.

Wenn diese Alkoxy-Silane bzw. ihre Hydrolyse- bzw. Kondensationsprodukte auf keratinischem Material angewendet werden, bildet sich auf dem Keratinmaterial ein Film oder auch ein Coating aus, welches das Keratinmaterial vollständig umhüllt und auf diese Weise die Eigenschaften des Keratinmaterials stark beeinflusst. Mögliche Anwendungsbereiche sind beispielsweise das permanente Styling oder auch die permanente Formveränderung von Keratinfasern. Hierbei werden die Keratinfasern mechanisch in die gewünschte Form gebracht und in dieser Form dann durch Ausbildung des vorbeschriebenen Coatings fixiert. Eine weitere ganz besonders gut geeignete Anwendungsmöglichkeit ist die Färbung von Keratinmaterial; im Rahmen dieser Anwendung wird das Coating bzw. der Film in Gegenwart einer farbgebenden Verbindung, zum Beispiel eines Pigments, erzeugt. Der durch das Pigment gefärbte Film verbleibt auf dem Keratinmaterial bzw. den Keratinfasern und resultiert in überraschend waschbeständigen Färbungen.

Der große Vorteil des auf Alkoxy-Silanen basierenden Färbeprinzips liegt darin, dass die hohe Reaktivität dieser Verbindungsklasse ein sehr schnelles Coating ermöglicht. So können bereits nach sehr kurzen Anwendungszeiträumen von nur wenigen Minuten extrem gute Färbeergebnisse erzielt werden. Neben diesen Vorteilen birgt die hohe Reaktivität der Alkoxy-Silane jedoch auch einige Nachteile.

Aufgrund ihrer hohen Reaktivität können die organischen Alkoxy-Silane nicht zusammen mit größeren Wassermengen konfektioniert werden, da ein großer Überschuss an Wasser die sofortige Hydrolyse und im Anschluss daran eine Polymerisation initiiert. Die bei Lagerung der Alkoxy-Silane in wässrigem Medium stattfindende Polymerisierung äußert sich in einer Verdickung oder Gelierung der wässrigen Zubereitung. Hierdurch werden die Zubereitungen so hochviskos, gelig bzw. gallertartig, dass sie nicht mehr gleichmäßig auf dem Keratinmaterial aufgetragen werden können. Zudem ist die Lagerung der Alkoxy-Silane in Gegenwart hoher Wassermengen mit einem Verlust ihrer Reaktivität verbunden, so dass auch die Ausbildung eines widerstandsfähigen Coatings auf dem Keratinmaterial nicht mehr möglich ist.

Aus diesen Gründen ist es notwendig, die organischen Alkoxy-Silane in wasserfreiem bzw. wasserarmem Milieu zu lagern und die entsprechenden Zubereitungen in einem separaten Container zu konfektionieren. Aufgrund ihrer hohen Reaktivität können die Alkoxy-Silane nicht nur mit Wasser, sondern auch mit anderen kosmetischen Inhaltsstoffen reagieren. Zur Vermeidung aller unerwünschten Reaktionen enthalten die Zubereitungen mit Alkoxy-Silanen deshalb bevorzugt keine weiteren Inhaltsstoffe oder nur die ausgewählten Inhaltsstoffe, die sich gegenüber den Alkoxy-Silanen als chemisch inert herausgestellt haben. Die Konzentration der Alkoxy-Silane in der Zubereitung wird dementsprechend bevorzugt verhältnismäßig hoch gewählt. Die wasserarmen Zubereitungen, welche die Alkoxy-Silane in relativ hohen Konzentrationen enthalten, können auch als "Silan-Blend" bezeichnet werden.

Für die Applikation auf dem Keratinmaterial muss der Anwender nun diesen verhältnismäßig hoch konzentrierten Silan-Blend in eine anwendungsbereite Mischung überführen. In dieser anwendungsbereiten Mischung ist zum einen die Konzentration der organischen Alkoxy-Silane erniedrigt, und zum anderen enthält die Anwendungsmischung auch einen höheren Anteil an Wasser (bzw. einem alternativen Inhaltsstoff), durch welchen die zum Coating führende Polymerisation ausgelöst wird.

Es hat sich als extrem große Herausforderung herausgestellt, die Polymerisationsgeschwindigkeit, d.h. die Geschwindigkeit, mit der sich das Coating auf dem Keratinmaterial ausbildet, optimal an die Anwendungsbedingungen anzupassen.

Bei Anwendung auf menschlichen Haaren führt eine zu schnelle Polymerisationsgeschwindigkeit beispielsweise dazu, dass die Polymerisation bereits abgeschlossen ist, bevor alle Haarpartien behandelt werden konnten. Eine zu schnelle Polymerisation macht deshalb die Ganzkopfbehandlung unmöglich. Im Färbeprozess äußert sich die zu schnelle Polymerisation in einem extrem ungleichmäßigen Farbergebnis, und die Haarpartien, die zuletzt behandelt wurden, werden nur mangelhaft gefärbt.

Bei einer zu langsam ablaufenden Polymerisation können andererseits zwar alle Areale des Haares ohne Zeitdruck behandelt werden, jedoch steigt hierdurch die Anwendungsdauer. Bei zu langsamer Polymerisation kommt daher der große Vorteil dieser Färbetechnologie, die Ausbildung von waschechten Färbungen innerhalb kürzester Anwendungszeiträume, nicht zum Tragen.

Es war die Aufgabe der vorliegenden Anmeldung, ein Verfahren zum Behandeln von keratinischem Material aufzufinden, mittels welchem die Polymerisationsgeschwindigkeit der organischen Alkoxy-silane an die Anwendungsbedingungen, insbesondere an die bei Anwendung auf dem menschlichen Kopf herrschenden Bedingungen, angepasst werden konnte. Mit anderen Worten wurde nach einem Verfahren gesucht, durch welches die organischen Alkoxy-silane so lange reaktiv bleiben, dass eine Ganzkopfbehandlung ermöglicht wird, ohne den Anwendungszeiträum über Gebühr zu verlängern.

Überraschenderweise hat sich herausgestellt, dass diese Aufgabe in vollem Umfang gelöst werden kann, wenn das Keratinmaterial in einem Verfahren behandelt wird, bei dem zwei Zusammensetzungen (A) und (B) auf dem Keratinmaterial angewendet werden. Bei der ersten Zusammensetzung (A) handelt es sich um den zuvor beschriebenen wasserarmen Silan-Blend. Die zweite Zusammensetzung (B) ist wasserhaltig und enthält zudem mindestens eine speziellea aromatische Verbindung der Formel (AR-I). Bei der Anwendung kommen beide Zusammensetzungen (A) und (B) miteinander in Kontakt, wobei dieser Kontakt entweder durch vorheriges Abmischen von (A) und (B) entsteht.

Ein erster Gegenstand der vorliegenden Erfindung ist ein Verfahren zum Behandeln von keratinischem Material, insbesondere menschlichen Haaren, bei dem auf dem keratinischen Material eine Zusammensetzung angewendet wird, die unmittelbar vor der Anwendung durch Vermischen einer ersten Zusammensetzung (A) und einer zweiten Zusammensetzung (B) hergestellt wurde, wobei
- die erste Zusammensetzung (A)- bezogen auf das Gesamtgewicht der Zusammensetzung (A) - enthält
   (A1) weniger als 10 Gew.-% Wasser und
   (A2) mindestens ein organisches C₁-C₆-Alkoxy-Silan das ausgewählt ist aus der Gruppe aus
      - (3-Aminopropyl)triethoxysilan
      - (3-Aminopropyl)trimethoxysilan
      - (2-Aminoethyl)triethoxysilan
      - (2-Aminoethyl)trimethoxysilan
      - (3-Dimethylaminopropyl)triethoxysilan
      - (3-Dimethylaminopropyl)trimethoxysilan
      - (2-Dimethylaminoethyl)triethoxysilan,
      - (2-Dimethylaminoethyl)trimethoxysilan
         und/oder deren Kondensationsprodukten, und
         mindestens ein organisches C₁-C₆-Alkoxysilan, das ausgewählt ist aus der Gruppe aus
      - Methyltrimethoxysilan
      - Methyltriethoxysilan
      - Ethyltrimethoxysilan
      - Ethyltriethoxysilan
      - Hexyltrimethoxysilan
      - Hexyltriethoxysilan
      - Octyltrimethoxysilan
      - Octyltriethoxysilan
      - Dodecyltrimethoxysilan,
      - Dodecyltriethoxysilan,
      und/oder deren Kondensationsprodukten, und
- die zweite Zusammensetzung (B), enthält
   (B1) Wasser und
   (B2) eine oder mehrere aromatische Verbindungen der Formel (AR-I) 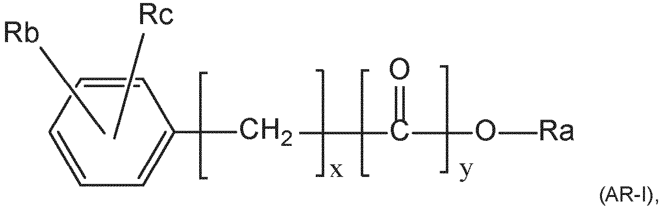 wobei
      - x: für eine ganze Zahl von 0 bis 3 steht,
      - y: für die Zahl 0 oder 1 seht,
      - Rₐ: für ein Wasserstoffatom, eine C₁-C₆-Alkylgruppe oder für eine Hydroxy-C₁-C₆-Alkylgruppe steht
      - R_{b,} Rc: unabhängig voneinander für für ein Wasserstoffatom, eine C₁-C₆-Alkylgruppe, eine Hydroxygruppe, ein Halogenatom aus der Gruppe Chlor, Brom, Fluor oder Jod, oder für eine C₁-C₆-Alkoxygruppe stehen.

Es hat sich gezeigt, dass die in der wasserhaltigen Zusammensetzung (B) enthaltenen speziellen aromatischen Verbindungen (B2) der Formel (AR-I) bei Kontakt mit der Zusammensetzung (A) die Polymerisationsgeschwindigkeit der organischen C₁-C₆-Alkoxy-Silane (A2) reduzieren. Überraschenderweise konnte die Reaktivität der organischen C₁-C₆-Alkoxy-Silane (A2) so optimal an die bei einem Ganzkopf-Haarfärbeprozess herrschenden Anwendungsbedingungen angepasst werden.

Bei Einsatz der beiden Zusammensetzungen (A) und (B) in einem Färbeverfahren auf Keratinmaterial, insbesondere auf menschlichen Haaren, konnten auf diese Weise Färbungen mit besonders hoher Gleichmäßigkeit erzeugt werden.

### Behandlung von keratinischem Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

Unter Mitteln zur Behandlung von keratinischem Material werden beispielsweise Mittel zur Färbung des Keratinmaterials, Mittel zur Umformung oder Formgebung von keratinischem Material, insbesondere keratinischen Fasern, oder auch Mittel zur Konditionierung bzw. zur Pflege des keratinischen Materials verstanden. Besonders gute Eignung zeige die über das erfindungsgemäße Verfahren hergestellten Mittel zur Färbung von keratinischem Material, insbesondere zur Färbung von keratinischen Fasern, bei denen es sich besonders bevorzugt um menschliche Haare handelt.

Der Begriff "Mittel zur Färbung" wird im Rahmen dieser Erfindung für eine durch Einsatz von farbgebenden Verbindungen, wie beispielsweise thermochromen und photochromen Farbstoffen, Pigmenten, Mica, direktziehenden Farbstoffen und/oder Oxidationsfarbstoffen hervorgerufene Farbgebung des Keratinmaterials, insbesondere des Haares, verwendet. Bei dieser Färbung lagern sich die vorgenannten farbgebenden Verbindungen in einem besonders homogenen und glatten Film an der Oberfläche des Keratinmaterials ab oder diffundieren in die Keratinfaser hinein. Der Film bildet sich *in situ* durch Oligomerisierung bzw. Polymerisierung des oder der organischen Alkoxy-Silane, und durch die Wechselwirkung von farbgebender Verbindung und organischer Siliciumverbindung und optional weiteren Bestandteilen, wie beispielsweise einem filmbildenden, Polymer.

### Wassergehalt (A1) in der Zusammensetzung (A)

Das erfindunngsgemäße Verfahren ist gekennzeichnet durch die Anwendung einer ersten Zusammensetzung (A) auf dem keratinischen Material.

Zur Gewährleistung einer ausreichend hohen Lagerstabilität ist die Zusammensetzung (A) dadurch gekennzeichnet, dass die wasserarm, bevorzugt im wesentlichen wasserfrei, ist. Deshalb enthält die Zusammensetzung (A) - bezogen auf das Gesamtgewicht der Zusammensetzung (A) - weniger als 10 Gew.-% Wasser.

Bei einem Wassergehalt von knapp unter 10 Gew.-% sind die Zusammensetzungen (A) über längere Zeiträume lagerstabil. Um die Lagerstabilität noch weiter zu verbessern und um eine ausreichend hohe Reaktivität der organische C₁-C₆-Alkoxy-Silane (A2) zu gewährleisten, hat es sich jedoch als besonders bevorzugt herausgestellt, den Wassergehalt in der Zusammensetzung (A) noch weiter herabzusenken. Aus diesem Grund enthält erste Zusammensetzung (A) - bezogen auf das Gesamtgewicht der Zusammensetzung (A) - bevorzugt 0,01 bis 9,5 Gew.-%, weiter bevorzugt 0,01 bis 8,0 Gew.-%, noch weiter bevorzugt 0,01 bis 6,0 und ganz besonders bevorzugt 0,01 bis 4,0 Gew.-% Wasser (A1).

Im Rahmen einer ganz besonders bevorzugten Ausführungsform ist ein erfindungsgemäßes Verfahren dadurch gekennzeichnet, dass die erste Zusammensetzung (A) - bezogen auf das Gesamtgewicht der Zusammensetzung (A) - 0,01 bis 9,5 Gew.-%, bevorzugt 0,01 bis 8,0 Gew.-%, weiter bevorzugt 0,01 bis 6,0 und ganz besonders bevorzugt 0,01 bis 4,0 Gew.-% Wasser (A1) enthält.

### Organische C₁-C₆-Alkoxy-Silane (A2) und/oder deren Kondensationsprodukte in der Zusammensetzung (A)

Die Zusammensetzung (A) ist dadurch gekennzeichnet, dass sie die organischen C₁-C₆-Alkoxy-Silane (A2) und/oder deren Kondensationsprodukte enthält.

Bei den organischen C₁-C₆-Alkoxy-Silanen handelt es sich um organische, nicht polymere Siliciumverbindungen, die aus der Gruppe der Silane mit einem, Siliciumatom ausgewählt sind

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die Wasserstoff-Atome ganz oder teilweise durch organische Gruppen wie beispielsweise (substituierte) Alkylgruppen und/oder Alkoxygruppen ersetzt.

Kennzeichnend für die erfindungsgemäßen C₁-C₆-Alkoxy-Silane ist, dass mindestens eine C₁-C₆-Alkoxygruppe direkt an ein Siliciumatom gebunden vorliegt. Die erfindungsgemäßen C₁-C₆-Alkoxy-Silane umfassen damit mindestens eine Struktureinheit R'R"R‴Si-O-(C₁-C₆-Alkyl) wobei die Reste R', R" und R‴ für die drei übrigen Bindungsvalenzen des Siliciumatoms stehen.

Das oder diese an das Siliciumatom gebundenen C₁-C₆-Alkoxygruppen sind sehr reaktiv und werden in Anwesenheit von Wasser mit hoher Geschwindigkeit hydrolysiert, wobei die Reaktionsgeschwindigkeit unter anderem auch von der Anzahl der hydrolysierbaren Gruppen pro Molekül abhängt. Handelt es sich bei der hydrolysierbaren C₁-C₆-Alkoxy-Gruppe um eine Ethoxygruppe, so enthält die organische Siliciumverbindung bevorzugt eine Struktureinheit R'R"R‴Si-O-CH2-CH3. Die Reste R', R" und R‴ stellen wieder die drei übrigen freien Valenzen des Siliciumatoms dar.

Bereits der Zusatz geringer Wassermengen führt zunächst zur Hydrolyse und dann zu einer Kondensationsreaktion der organischen Alkoxy-silane untereinander. Aus diesem Grund können sowohl die organischen Alkoxy-silane (A2) als auch deren Kondensationsprodukte in der Zusammensetzung enthalten sein.

Unter einem Kondensationsprodukt wird ein Produkt verstanden, dass durch Reaktion von mindestens zwei organischen C₁-C₆-Alkoxy-Silanen unter Abspaltung von Wasser und/oder unter Abspaltung von einem C₁-C₆-Alkanol entsteht.

Die Kondensationsprodukte können beispielsweise Dimere, aber auch Trimere oder Oligomere sein, wobei die Kondensationsprodukte mit den Monomeren im Gleichgewicht stehen.

Abhängig von der eingesetzten bzw. in der Hydrolyse verbrauchten Wassermenge verschiebt sich das Gleichgewicht von monomerem C₁-C₆-Alkoxysilan zu Kondensationsprodukt.

Zur Lösung der erfindungsgemäßen Aufgabenstellung besonders gut geeignete organische Silicumverbindungen sind
- (3-Aminopropyl)triethoxysilan 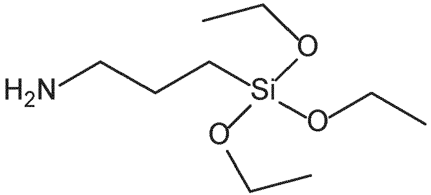
- (3-Aminopropyl)trimethoxysilan 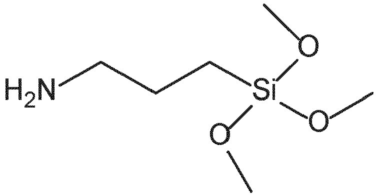
- (2-Aminoethyl)triethoxysilan 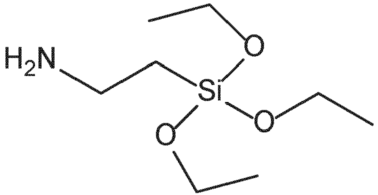
- (2-Aminoethyl)trimethoxysilan 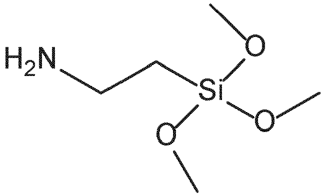
- (3-Dimethylaminopropyl)triethoxysilan 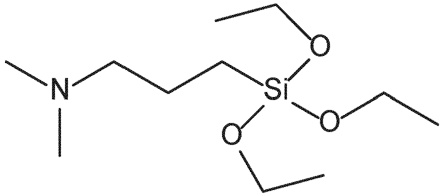
- (3-Dimethylaminopropyl)trimethoxysilan 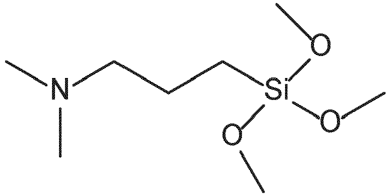
- (2-Dimethylaminoethyl)triethoxysilan. 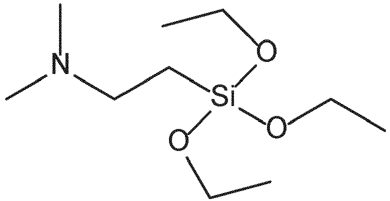
- (2-Dimethylaminoethyl)trimethoxysilan und/oder

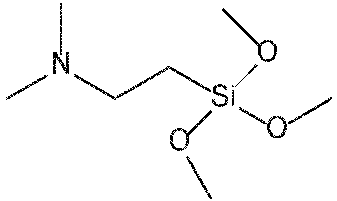

Das erfindungsgemäße Verfahren ist dadurch gekennzeichnet, dass das die erste Zusammensetzung (A) mindestens ein organisches C₁-C₆-Alkoxysilan (A2) enthält, das ausgewählt ist aus der Gruppe aus
- (3-Aminopropyl)triethoxysilan
- (3-Aminopropyl)trimethoxysilan
- (2-Aminoethyl)triethoxysilan
- (2-Aminoethyl)trimethoxysilan
- (3-Dimethylaminopropyl)triethoxysilan
- (3-Dimethylaminopropyl)trimethoxysilan
- (2-Dimethylaminoethyl)triethoxysilan,
- (2-Dimethylaminoethyl)trimethoxysilan
und/oder deren Kondensationsprodukten.

Die vorgenannten organische Siliciumverbindung der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erwerblich.

In weiteren Färbeversuchen hat es sich ebenfalls als ganz besonders vorteilhaft herausgestellt, wenn im erfindungsgemäßen Verfahren mindestens ein organisches C₁-C₆-Alkoxy-Silan (A2) aus der nachfolgenden Gruppe eingesetzt wurde
- Methyltrimethoxysilan 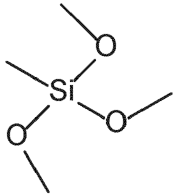
- Methyltriethoxysilan 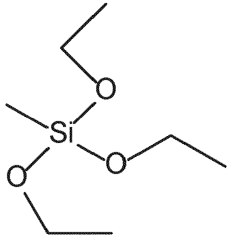
- Ethyltrimethoxysilan 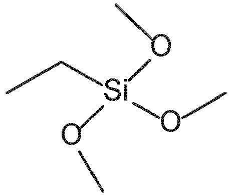
- Ethyltriethoxysilan 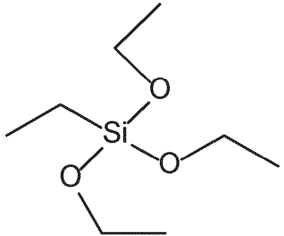
- n-Hexyltrimethoxysilan (auch bezeichnet als Hexyltrimethoxysilan) 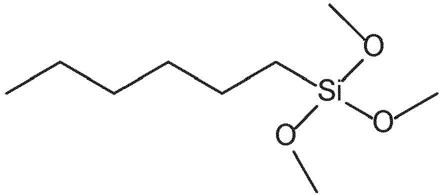
- n-Hexyltriethoxysilan (auch bezeichnet als Hexyltriethoxysilan) 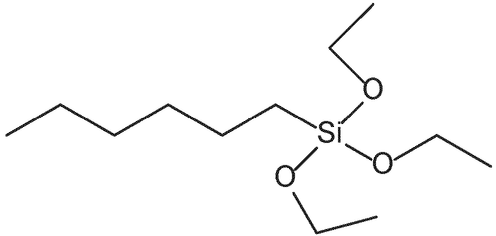
- n-Octyltrimethoxysilan (auch bezeichnet als Octyltrimethoxysilan) 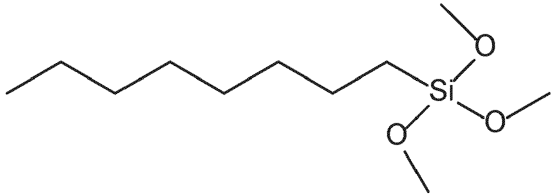
- n-Octyltriethoxysilan (auch bezeichnet als Octyltriethoxysilan) 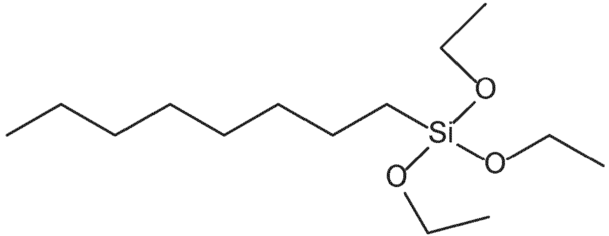
- n-Dodecyltrimethoxysilan (auch bezeichnet als Dodecyltrimethoxysilan) und/oder 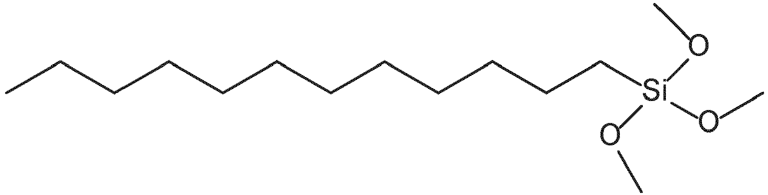
- n-Dodecyltriethoxysilan (auch bezeichnet als Dodecyltriethoxysilan).

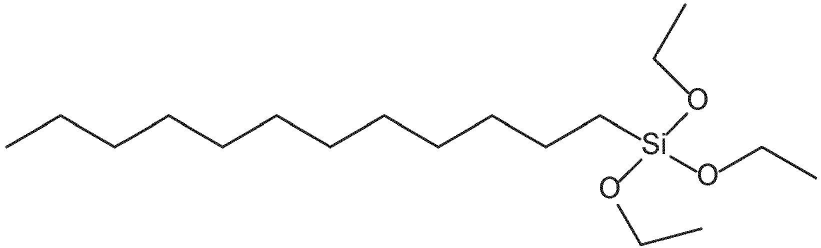

Das erfindungsgemäße Verfahren ist dadurch gekennzeichnet, dass die erste Zusammensetzung (A) mindestens ein organisches C₁-C₆-Alkoxysilan (A2) enthält, das ausgewählt ist aus der Gruppe aus
- Methyltrimethoxysilan
- Methyltriethoxysilan
- Ethyltrimethoxysilan
- Ethyltriethoxysilan
- Hexyltrimethoxysilan
- Hexyltriethoxysilan
- Octyltrimethoxysilan
- Octyltriethoxysilan
- Dodecyltrimethoxysilan,
- Dodecyltriethoxysilan,
und/oder deren Kondensationsprodukten.

Bei den entsprechenden Hydrolyse bzw. Kondensationsprodukten handelt es sich beispielsweise um die folgenden Verbindungen:
Hydrolyse von C₁-C₆-Alkoxy-Silan der Formel (S-I) mit Wasser (Reaktionsschema am Beispiel von 3-Aminopropyltriethoxysilan):

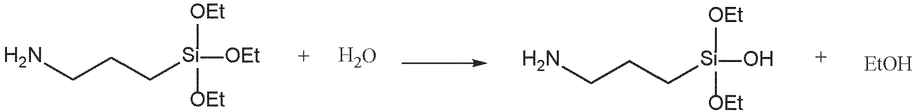

In Abhängigkeit von der eingesetzten Menge an Wasser kann die Hydrolyse-Reaktion auch mehrfach pro eingesetztem C₁-C₆-Alkoxy-Silan stattfinden: 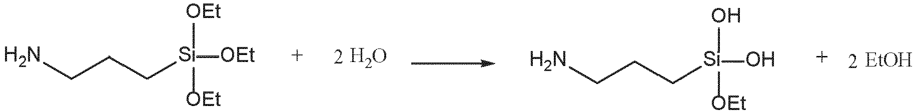 bzw.

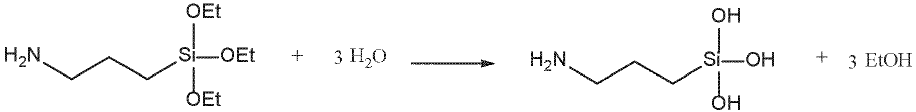

Hydrolyse von C₁-C₆-Alkoxy-Silan der Formel (S-IV) mit Wasser (Reaktionsschema am Beispiel von Methyltrimethoxysilan):

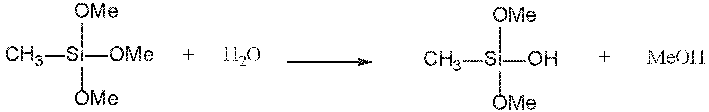

In Abhängigkeit von der eingesetzten Menge an Wasser kann die Hydrolyse-Reaktion auch mehrfach pro eingesetztem C₁-C₆-Alkoxy-Silan stattfinden: 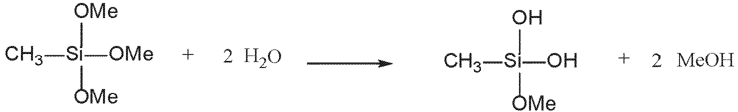 bzw.

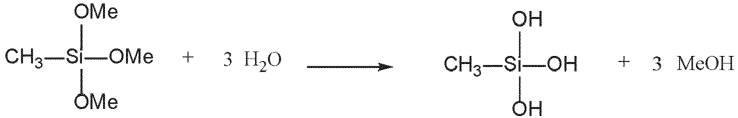

Mögliche Kondensationsreaktionen sind beispielsweise (gezeigt anhand des Gemisches (3-Aminopropyl)triethoxysilan und Methyltrimethoxysilan): 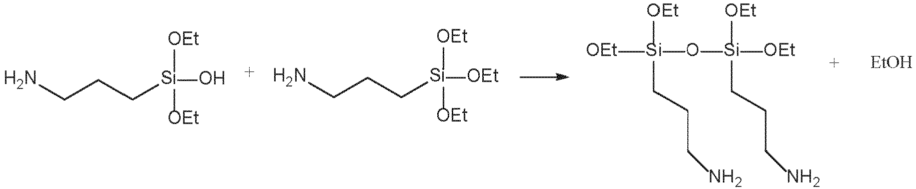 und/oder 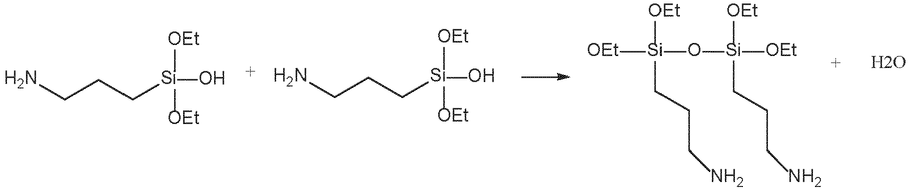 und/oder 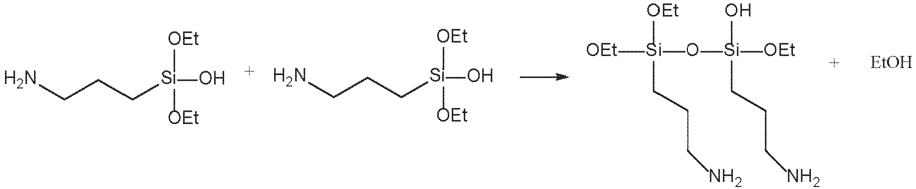 und/oder 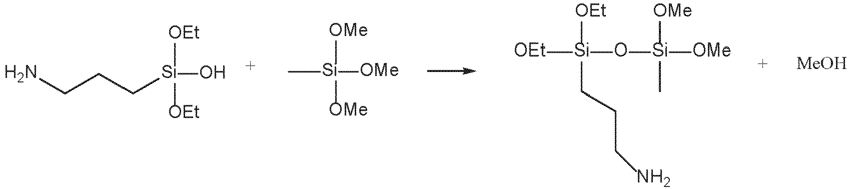 und/oder 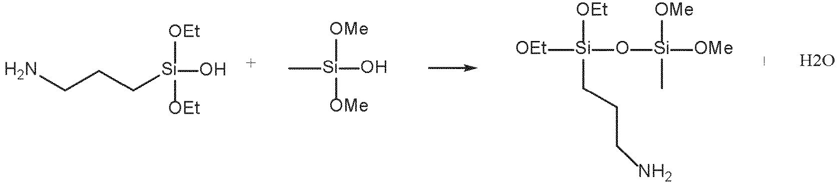 und/oder 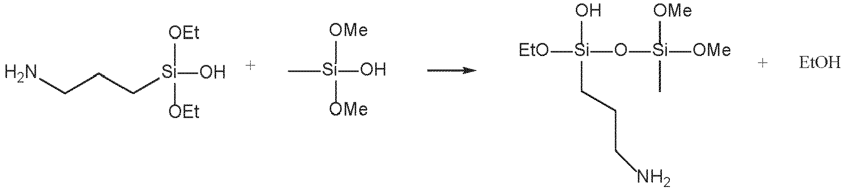 und/oder

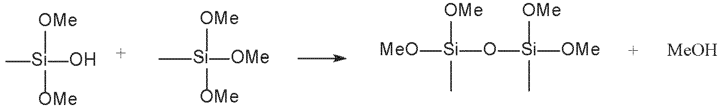

In den obigen beispielhaften Reaktionsschemata ist jeweils die Kondensation zu einem Dimer gezeigt, jedoch sind auch weitergehende Kondensationen zu Oligomeren mit mehreren Silan-Atomen möglich und auch bevorzugt.

Die erfindungsgemäße Zusammensetzung (A) kann die organischen C₁-C₆-Alkoxysilane (A2) in verschiedenen Mengenanteilen enthalten. Diese bestimmt der Fachmann in Abhängigkeit von der gewünschten Dicke des Silan-Coatings auf dem Keratinmaterial und von der Menge des zu behandelnden Keratinmaterials.

Besonders lagerstabile Zubereitungen mit sehr gutem Färberesultat bei der Anwendung konnten erhalten werden, wenn die Zusammensetzung (A) - bezogen auf ihr Gesamtgewicht -die organischen C₁-C₆-Alkoxysilane (A2) und/oder die Kondensationsprodukte hiervon in einer Gesamtmenge von 30,0 bis 85,0 Gew.-%, bevorzugt von 35,0 bis 80,0 Gew.-%, weiter bevorzugt von 40,0 bis 75,0 Gew.-%, noch weiter bevorzugt von 45,0 bis 70,0 Gew.-% und ganz besonders bevorzugt von 50,0 bis 65,0 Gew.-% enthält.

In einer weiteren Ausführungsform ist ein ganz besonders bevorzugtes Verfahren dadurch gekennzeichnet, dass die erste Zusammensetzung (A) - bezogen auf das Gesamtgewicht der Zusammensetzung (A) - die organischen C₁-C₆-Alkoxysilane (A2) und/oder die Kondensationsprodukte hiervon in einer Gesamtmenge von 30,0 bis 85,0 Gew.-%, bevorzugt von 35,0 bis 80,0 Gew.-%, weiter bevorzugt von 40,0 bis 75,0 Gew.-%, noch weiter bevorzugt von 45,0 bis 70,0 Gew.-% und ganz besonders bevorzugt von 50,0 bis 65,0 Gew.-% enthält.

### Weitere kosmetische Inhaltsstoffe in der Zusammensetzung (A)

Prinzipiell kann die Zusammensetzung (A) auch noch einen oder mehrere weitere kosmetische Inhaltsstoffe enthalten.

Bei den fakultativ in der Zusammensetzung (A) einsetzbaren kosmetischen Inhaltsstoffen kann es sich um alle geeigneten Bestandteile handeln, um dem Mittel weitere positive Eigenschaften zu verleihen. Beispielsweise können in der Zusammensetzung (A) ein Lösungsmittel, ein verdickendes oder filmbildendes Polymer, eine oberflächenaktive Verbindung aus der Gruppe der nichtionischen, kationischen, anionischen oder zwitterionischen/amphoteren Tenside, der farbgebenden Verbindungen aus der Gruppe der Pigmente, der direktziehenden Farbstoffe, der Oxidationsfarbstoffvorprodukte, der Fettkomponenten aus der Gruppe der C₈-C₃₀-Fettalkohole, der Kohlenwasserstoffverbindungen, Fettsäureester, der zur Gruppe der pH-Regulatoren zugehörigen Säuren und Basen, der der Parfüme, der Konservierungsmittel, der Pflanzenextrakte und der Proteinhydrolysate enthalten sein.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen.

Wie bereits zuvor beschrieben, können die organischen C₁-C₆-Alkoxysilane (A2) jedoch nicht nur mit Wasser, sondern auch mit anderen kosmetischen Inhaltsstoffen reagieren. Zur Vermeidung dieser unerwünschten Reaktionen enthalten die Zubereitungen (A) mit Alkoxy-Silanen deshalb bevorzugt keine weiteren Inhaltsstoffe oder nur die ausgewählten Inhaltsstoffe, die sich gegenüber den Alkoxy-Silanen als chemisch inert herausgestellt haben. Als ganz besonders bevorzugt hat es sich in diesem Zusammenhang erwiesen, in der Zusammensetzung (A) einen kosmetischen Inhaltsstoff einzusetzen aus der Gruppe aus Hexamethyldisiloxan, Octamethyltrisiloxan, Decamethyltetrasiloxan, Hexamethylcyclotrisiloxan, Octamethylcyclotetrasiloxan und/oder Decamethylcyclopentasiloxan einzusetzen.

In einer weiteren ganz besonders bevorzugten Ausführungsform ist ein erfindungsgemäßes Verfahren dadurch gekennzeichnet, das die erste Zusammsensetzung (A) mindestens einen kosmetischen Inhaltsstoff aus der Gruppe aus Hexamethyldisiloxan. Octamethyltrisiloxan, Decamethyltetrasiloxan, Hexamethylcyclotrisiloxan, Octamethylcyclotetrasiloxan und Decamethylcyclopentasiloxan enthält.

Hexamethyldisiloxan besitzt die CAS-Nummer 107-46-0 und kann beispielsweise bei Sigma-Aldrich kommerziell erworben werden.

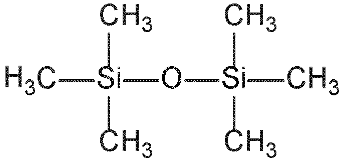

Octamethyltrisiloxan besitzt die CAS-Nummer 107-51-7 und ist ebenfalls bei Sigma-Aldrich kommerziell erhältlich.

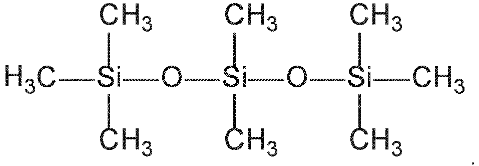

Decamethyltetrasiloxan trägt die CAS-Nummer 141-62-8 und ist ebenfalls bei Sigma-Aldrich kommerziell erhältlich.

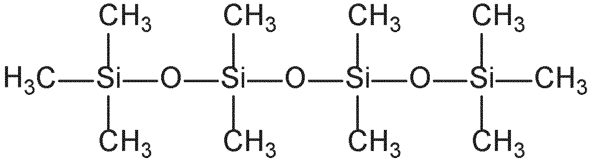

Hexamethylcyclotrisiloxan besitzt die CAS-Nr. 541-05-9.

Octamethylcyclotetrasiloxan besitzt die CAS-Nr. 556-67-2.

Decamethylcyclopentasiloxan besitzt die CAS-Nr. 541-02-6.

Als ganz besonders bevorzugt hat sich der Einsatz von Hexamethyldisiloxan in der Zusammensetzung (A) herausgestellt. Besonders bevorzugt ist Hexamethyldisiloxan - bezogen auf das Gesamtgewicht der Zusammensetzung (A) - in Mengen von 10,0 bis 50,0 Gew.-%, bevorzugt 15,0 bis 45,0 Gew.-%, weiter bevorzugt 20,0 bis 40,0 Gew.-%, noch weiter bevorzugt 25,0 bis 35,0 Gew.-% und ganz besonders bevorzugt 31,0 bis 34,0 Gew.-% in der Zusammensetzung (A) enthalten.

In einer weiteren ganz besonders bevorzugten Ausführungsform ist ein erfindungsgemäßes Verfahren dadurch gekennzeichnet, dass die erste Zusammensetzung (A) - bezogen auf das Gesamtgewicht der Zusammensetzung (A) - 10,0 bis 50,0 Gew.-%, bevorzugt 15,0 bis 45,0 Gew.-%, weiter bevorzugt 20,0 bis 40,0 Gew.-%, noch weiter bevorzugt 25,0 bis 35,0 Gew.-% und ganz besonders bevorzugt 31,0 bis 34,0 Gew.-% Hexamethyldisiloxan enthält.

### Wassergehalt (B1) in der Zusammensetzung (B)

Kennzeichnend für das erfindungsgemäße Verfahren ist die Anwendung einer zweiten Zusammensetzung (B) auf dem keratinischen Material, insbesondere auf den menschlichen Haaren.

Bei der Anwendung auf dem keratinischen Material kommen die Zusammensetzungen (A) und (B) in Kontakt, wobei dieser Kontakt durch vorheriges Vermischen der beiden Zusammensetzungen (A) und (B) hergestellt wird. Durch das Vermischen von (A) und (B) wird das anwendungsbereite Keratinbehandlungsmittel hergestellt, d.h. der lagerstabile bzw. lagerfähige Silan-Blend (A) wird durch Kontakt mit (B) in seine reaktive Form überführt. Mit Vermischen der Zusammensetzungen (A) und (B) startet eine von den Alkoxy-Silan-Monomeren oder Alkoxy-Silan-Oligomeren ausgehende Polymerisationsreaktion, die schließlich zur Ausbildung des Films bzw. des Coatings auf dem Keratinmaterial führt.

Je mehr Wasser mit dem oder den organischen C₁-C₆-Alkoxy-Silanen in Kontakt kommt, in desto stärkerem Ausmaß läuft die Polymerisationsreaktion ab. Enthält die Zusammensetzung (B) beispielsweise sehr viel Wasser, so polymerisieren die zuvor in der wasserarmen Zusammensetzung (A) vorhandenen monomeren bzw. oligomeren Silan-Kondensate nun zu Polymeren höheren oder hohen Molekulargewichts. Die hochmolekularen Silan-Polymer bilden dann den Film auf dem keratinischen Material. Aus diesem Grund ist Wasser (B1) ein erfindungswesentlicher Inhaltsstoff der Zusammensetzung (B).

Durch die Menge an Wasser in der Zusammensetzung (B) kann die Polymerisationsgeschwindigkeit der organischen C₁-C₆-Alkoxy-Silane (A2) zum Zeitpunkt der Anwendung mitbestimmt werden. Um bei einer Haarfärbung auf dem gesamten Kopf ein gleichmäßiges Farbergebnis zu gewährleisten, sollte die Polymerisationsgeschwindigkeit, d.h. die Schnelligkeit, mit der sich das Coating ausbildet, jedoch auch nicht zu hoch sein. Aus diesem Grund hat es sich als ganz besonders bevorzugt herausgestellt, die Menge an Wasser in der Zusammensetzung (B) nicht zu hoch zu wählen.

Besonders gleichmäßige Färbungen auf dem gesamten Kopf konnten erhalten werden, wenn die Zusammensetzung (B) - bezogen auf das Gesamtgewicht der Zusammsentzung (B) - 0,1 bis 95,0 Gew.-%, bevorzugt 20,0 bis 95,0 Gew.-%, weiter bevorzugt 30,0 bis 95,0 Gew.-% , nocht weiter bevorzugt 50,0 bis 95,0 Gew.-% und ganz besonders bevorzugt 70,0 bis 95,0 Gew.-% Wasser (B1) enthält.

Im Rahmen einer weiteren ganz besonders bevorzugten Ausführungsform ist ein erfindungsgemäßes Verfahren dadurch gekennzeichnet, dass die zweite Zuammensetzung (B) - bezogen auf das Gesamtgewicht der Zusammsentzung (B) - 0,1 bis 95,0 Gew.-%, bevorzugt 20,0 bis 95,0 Gew.-%, weiter bevorzugt 30,0 bis 95,0 Gew.-% , noch weiter bevorzugt 50,0 bis 95,0 Gew.-% und ganz besonders bevorzugt 70,0 bis 95,0 Gew.-% Wasser (B1) enthält.

### Aromatische Verbindungen der Formel (AR-I) in der Zusammensetzung (B)

Kennzeichnend für die Zusammensetzung (B) ist weiterhin ihr Gehalt an mindestens einer aromatischen Verbindung (B2) der Formel (AR-I) 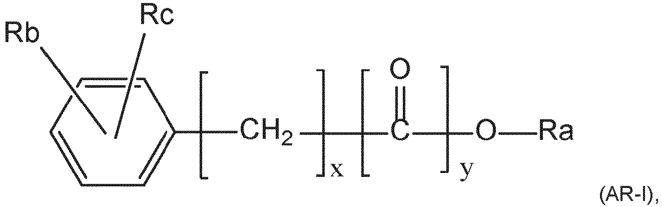 wobei
- x: für eine ganze Zahl von 0 bis 3 steht,
- y: für die Zahl 0 oder 1 seht,
- Rₐ: für ein Wasserstoffatom, eine C₁-C₆-Alkylgruppe oder für eine Hydroxy-C₁-C₆-Alkylgruppe steht
- R_{b,} Rc: unabhängig voneinander für für ein Wasserstoffatom, eine C₁-C₆-Alkylgruppe, eine Hydroxygruppe, ein Halogenatom aus der Gruppe Chlor, Brom, Fluor oder Jod oder für eine C₁-C₆-Alkoxygruppe stehen.

Überraschenderweise hat sich herausgestellt, dass der Einsatz mindestens eines speziellen Aromaten der Formel (AR-I) die Reaktionsgeschwindigkeit der organischen C₁-C₆-Alkoxy-silane so optimiert, dass eine gleichmäßige Färbung auf dem gesamten Kopf ermöglicht wird.

Die erfindungsgemäßen Aromaten der Formel (AR-I), insbesondere die bevorzugten und besonders bevorzugten Verbindungen, sind entweder hydrophobe Substanzen mit Alkyl- bzw. Alkenylkette(n), oder aber Substanzen, die neben einer Alkylkette auch noch ein oder mehrere Hydroxygruppen in ihrer Struktur tragen.

Hydrophobe Substanzen können in Gegenwart von Wasser unter Ausbildung von Mizell-Systemen Emulsionen formen. Ohne auf diese Theorie festgelegt zu sein, wird vermutet, dass die C₁-C₆-Alkoxysilane - entweder in Form ihrer Monomere oder gegebenenfalls in Form ihrer kondensierten Oligomere - in diese hydrophobe Umgebung bzw. in die Mizell-Systeme eingebettet werden, so dass sich die Polarität ihrer Umgebung verändert. Bedingt durch den hydrophoben Charakter der Aromaten (B2) wird auch die Umgebung der C₁-C₆-Alkoxysilane hydrophobiert. Es wird angenommen, dass die zum Film bzw. Coating führende Polymerisationsreaktion der C₁-C₆-Alkoxy-silane in hydrophobem Milieu mit reduzierter Geschwindigkeit abläuft.

Wenn die erfindungsgemäßen Aromaten protische Substanzen sind, so enthalten sie mindestens eine Hydroxy-Gruppe. In diesem Zusammenhang wird vermutet, dass die protischen Aromaten der Formel (AR-I) über ihre Hydroxy-Gruppe(n) auch mit den C₁-C₆-Alkoxysilanen reagieren können, die Reaktion zwischen protischem Aromaten (AR-I) und C₁-C₆-Alkoxysilanen jedoch langsamer abläuft als die analoge Reaktion zwischen Wasser und C₁-C₆-Alkoxysilanen. In Summe wird auf diesem Wege damit ebenfalls die Hydrolyse- und/oder die Kondensationsreaktion der C₁-C₆-Alkoxy-silane reduziert.

Die Substituenten Ra, Rb, Rc in den Verbindungen der Formel (AR-I) sind nachstehend beispielhaft erläutert:
Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; die Hydroxymethylgruppe und die 2-Hydroxyethylgruppe sind besonders bevorzugt.

Beispiele für eine C₁-C₆-Alkoxygruppe sind die Methoxygruppe, die Ethoxygruppe und die n-Propoxygruppe, wobei die Methoxygruppe und die Ethoxygruppe besonders bevorzugt sind.

Durch die Variation der Reste Ra, Rb und Rc sowie x und y kann die Polarität des Aromaten (AR-I) eingestellt werden und die Polymerisationsgeschwindigkeit der C₁-C₆-Alkoxysilane besonders gut an die jeweils gewählten Anwendungsbedingungen angepasst werden.

Bei den den aromatischen Verbindungen (B2) der Formel (AR-I) kann x für eine ganze Zahl von 0 bis 3 stehen. Im Rahmen einer Ausführungsform wurden ganz besonders gute Ergebnisse erhalten, wenn x für die Zahl 0 oder 1 steht.

Im Rahmen einer ganz besonders bevorzugten Ausführungsform ist ein erfindungsgemäßes Verfahren dadurch gekennzeichnet, dass die zweite Zusammensetzung (B) eine oder mehrere aromatische Verbindungen (B2) der Formel (AR-I) enthält,
wobei
x für die Zahl 0 oder 1 steht.

Der Rest y kann für die ganze Zahl von 0 oder 1 stehen. Im Rahmen dieser Ausführungsform ist es weiterhin ganz besonders bevorzugt, wenn
x für die Zahl 0 steht und y für die Zahl 1 steht oder
x für die Zahl 1 steht und y für die Zahl 0 steht oder
x für die Zahl 0 steht und y für die Zahl 0 steht.

Im Rahmen einer ganz besonders bevorzugten Ausführungsform ist ein erfindungsgemäßes Verfahren dadurch gekennzeichnet, dass die zweite Zusammensetzung (B) eine oder mehrere aromatische Verbindungen (B2) der Formel (AR-I) enthält,
wobei
x für die Zahl 0 steht und y für die Zahl 1 steht oder
x für die Zahl 1 steht und y für die Zahl 0 steht oder
x für die Zahl 0 steht und y für die Zahl 0 steht.

Im Hinblick auf die Anpassung der Reaktionsgeschwindigkeit wurden weiterhin besonders gute Ergebnisse erhalten, wenn in der Zusammensetzung (B) eine oder mehrere aromatische Verbindungen (B2) der Formel (AR-I) eingesetzt wurden, bei welchen der Rest Ra für ein Wasserstoffatom oder für eine eine C₁-C₆-Alkylgruppe, besonders bevorzugt für ein Wasserstoffatom, für eine n-Pentylgruppe, für eine n-Butylgruppe, für eine n-Propylgruppe, für eine Ethylgruppe oder für eine Methylgruppe steht.

Im Rahmen einer ganz besonders bevorzugten Ausführungsform ist ein erfindungsgemäßes Verfahren dadurch gekennzeichnet, dass die zweite Zusammensetzung (B) eine oder mehrere aromatische Verbindungen (B2) der Formel (AR-I) enthält, wobei
- Rₐ: für ein Wasserstoffatom oder für eine eine C₁-C₆-Alkylgruppe, besonders bevorzugt für ein Wasserstoffatom, für eine n-Pentylgruppe, für eine n-Butylgruppe, für eine n-Propylgruppe, für eine Ethylgruppe oder für eine Methylgruppe steht.

Expizit ganz besonders bevorzugt steht Ra für ein Wasserstoffatom oder für eine n-Pentylgruppe.

Weiterhin können die Reste Rb und Rc in den aromatischen Verbindungen der Formel (AR-I) unabhängig voneinander für ein Wasserstoffatom, eine C₁-C₆-Alkylgruppe, eine Hydroxygruppe, ein Halogenatom aus der Gruppe Chlor, Brom, Fluor oder Jod oder für eine C₁-C₆-Alkoxygruppe stehen.

Sehr gute Ergebnisse wurden erhalten, wenn Rb und Rc unabhängig voneinander für ein Wasserstoffatom, für eine Hydroxygruppe oder für eine C₁-C₆-Alkylgruppe stehen.

Ganz besonders gute Ergebnisse wurden erhalten, wenn Rb und Rc unabhängig voneinander für ein Wasserstoffatom oder für eine C₁-C₆-Alkylgruppe stehen.

Im Rahmen einer ganz besonders bevorzugten Ausführungsform ist ein erfindungsgemäßes Verfahren dadurch gekennzeichnet, dass die zweite Zusammensetzung (B) eine oder mehrere aromatische Verbindungen (B2) der Formel (AR-I) enthält, wobei
- Rb, Rc: unabhängig voneinander für ein Wasserstoffatom, für eine Hydroxygruppe oder für eine C₁-C₆-Alkylgruppe, ganz besonders bevorzugt für ein Wasserstoffatom oder für eine C₁-C₆-Alkylgruppe stehen.

Besonders bevorzugte aromatische Verbindungenn (B2) der Formel (AR-I) können ausgewählt sein aus der Liste aus
- Thymol (2-Isopropyl-5-methyl-phenol) 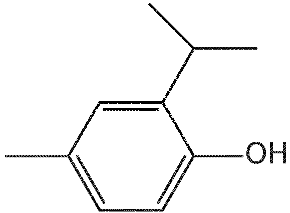
- Benzylalkohol 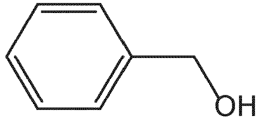
- Benzoesäure 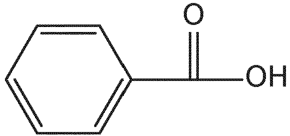
- Benzoesäuremethylester 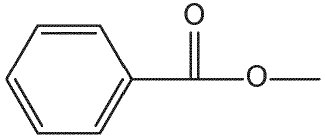
- Benzoesäure-ethylester 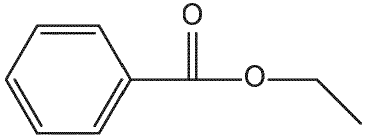
- Benzoesäure-n-propylester 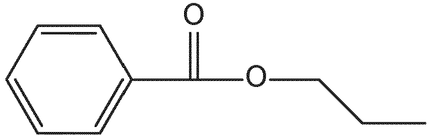
- Benzoesäure-isopropylester 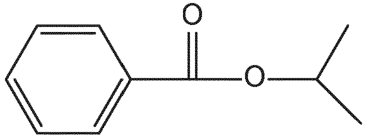
- Benzoesäure-n-butylester 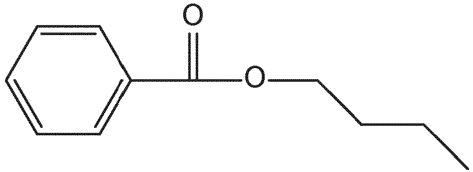
- Benzoesäure-n-pentylester 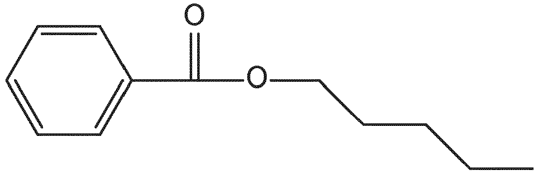
- Benzoesäure-n-hexylester 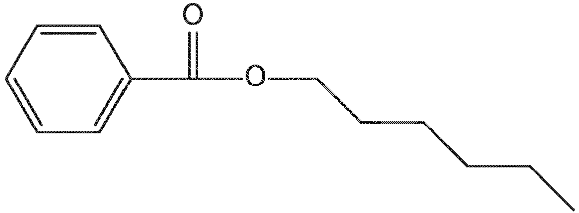
- 2-Hydroxybenzoesäure 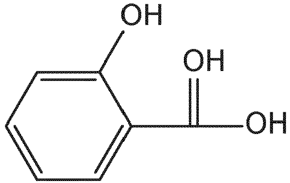
- 2-Hydroxybenzoesäure- methylester 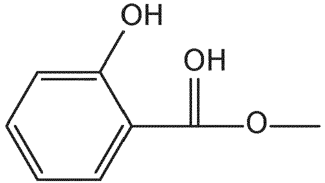
- 2-Hydroxybenzoesäure- ethylester 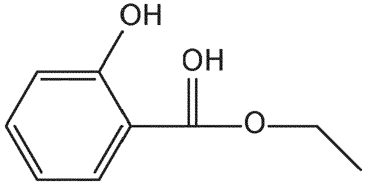
- 2-Hydroxybenzoesäure- n-propylester 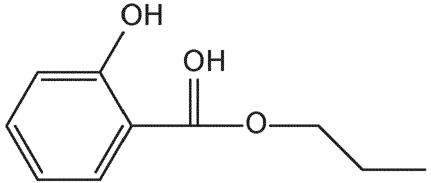
- 2-Hydroxybenzoesäure-isopropylester 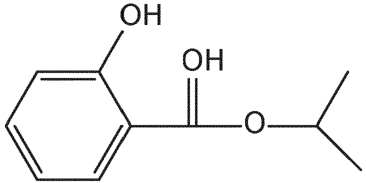
- 2-Hydroxybenzoesäure-n-butylester 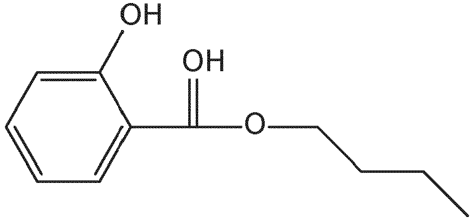
- 2-Hydroxybenzoesäure-n-pentylester 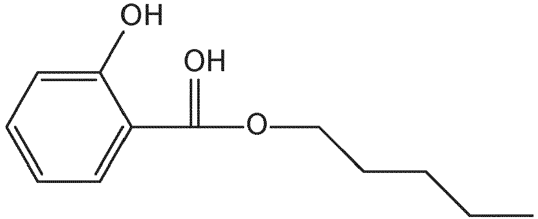
- 2-Hydroxybenzoesäure-n-hexylester 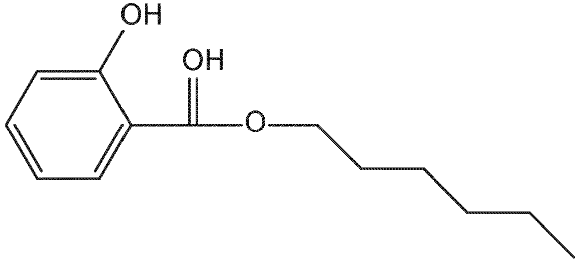
- 3-Hydroxybenzoesäure 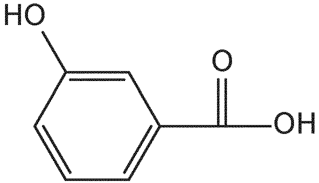
- 3-Hydroxybenzoesäure- methylester 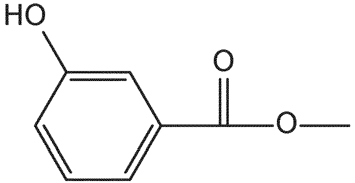
- 3-Hydroxybenzoesäure- ethylester 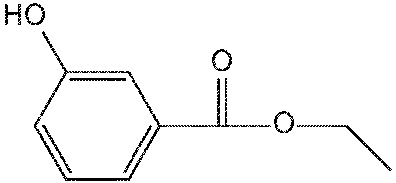
- 3-Hydroxybenzoesäure- n-propylester 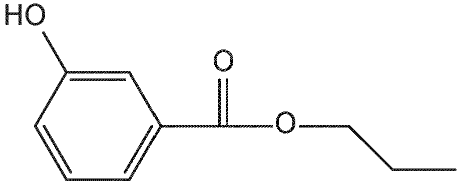
- 3-Hydroxybenzoesäure-isopropylester 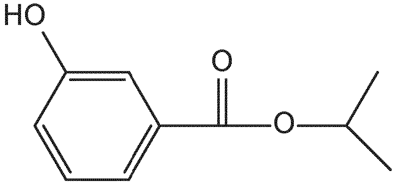
- 3-Hydroxybenzoesäure-n-butylester 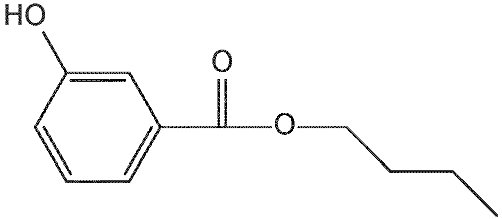
- 3-Hydroxybenzoesäure-n-pentylester 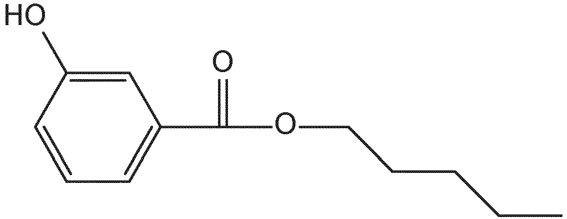
- 3-Hydroxybenzoesäure-n-hexylester 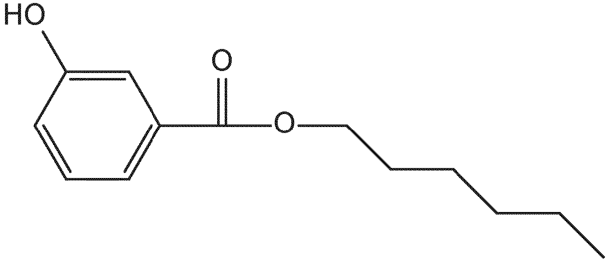
- 4-Hydroxybenzoesäure 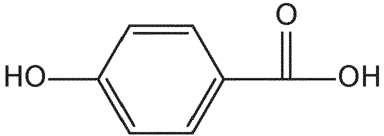
- 4-Hydroxybenzoesäure- methylester 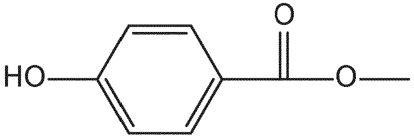
- 4-Hydroxybenzoesäure- ethylester 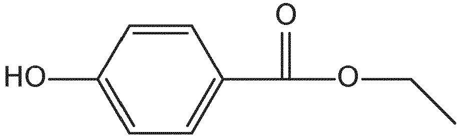
- 4-Hydroxybenzoesäure- n-propylester 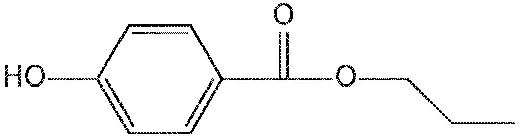
- 4-Hydroxybenzoesäure-isopropylester 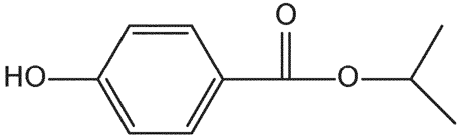
- 4-Hydroxybenzoesäure-n-butylester 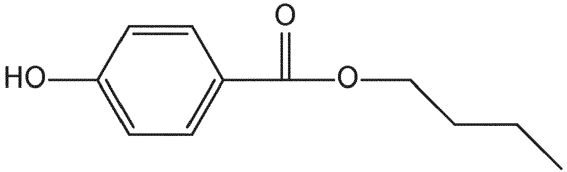
- 4-Hydroxybenzoesäure-n-pentylester 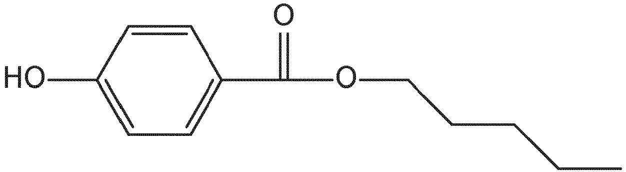
- 4-Hydroxybenzoesäure-n-hexylester 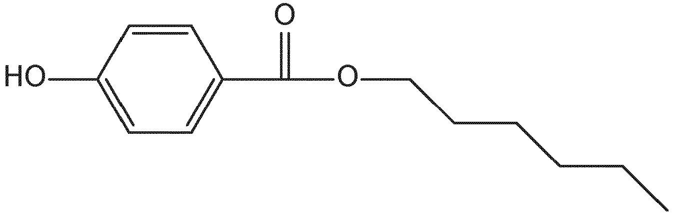
- 2-Methoxybenzoesäure 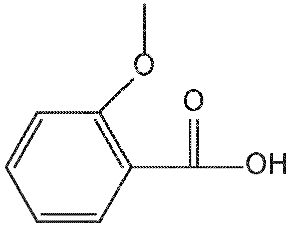
- 2-Methoxybenzoesäure- methylester 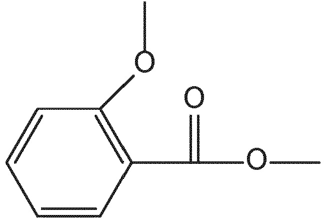
- 2-Methoxybenzoesäure- ethylester 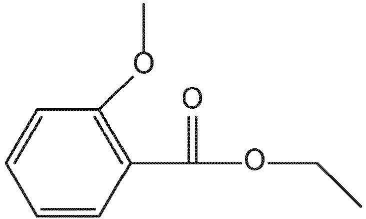
- 2-Methoxybenzoesäure- n-propylester 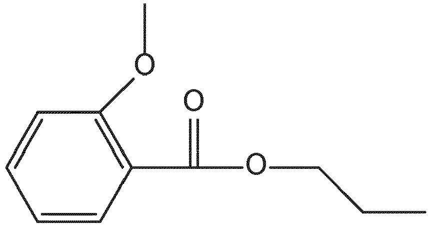
- 2-Methoxybenzoesäure-isopropylester 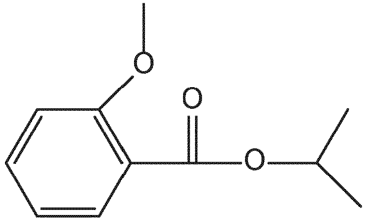
- 2-Methoxybenzoesäure-n-butylester 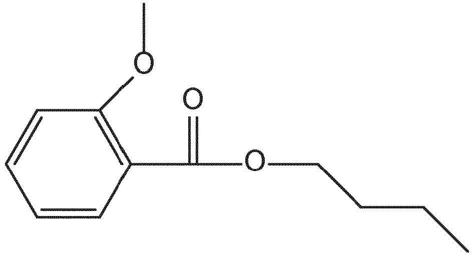
- 2-Methoxybenzoesäure-n-pentylester 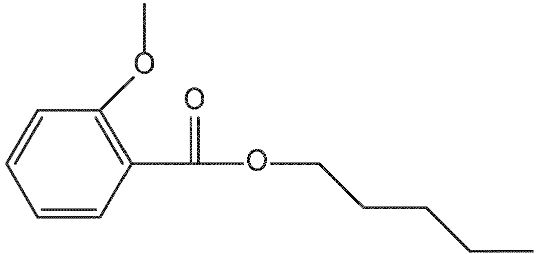
- 2-Methoxybenzoesäure-n-hexylester 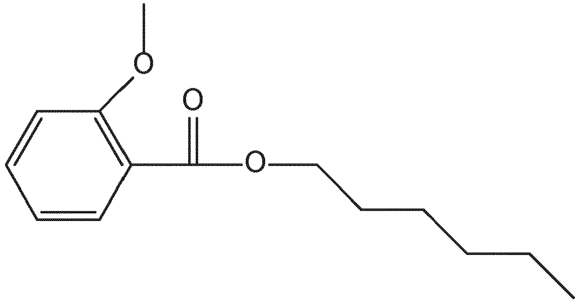
- 3-Methoxybenzoesäure 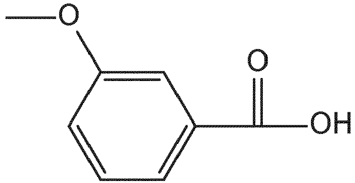
- 3-Methoxybenzoesäure- methylester 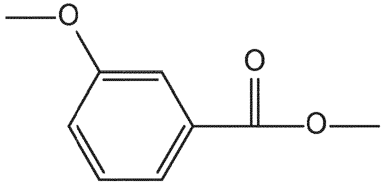
- 3-Methoxybenzoesäure- ethylester 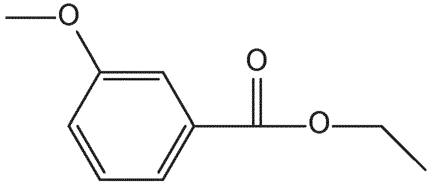
- 3-Methoxybenzoesäure- n-propylester 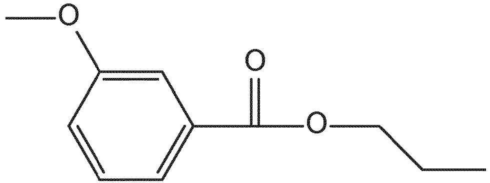
- 3-Methoxybenzoesäure-isopropylester 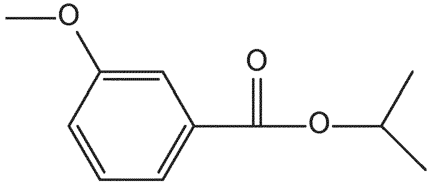
- 3-Methoxybenzoesäure-n-butylester 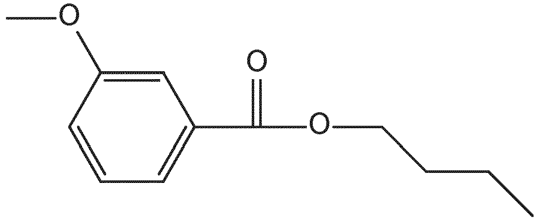
- 3-Methoxybenzoesäure-n-pentylester 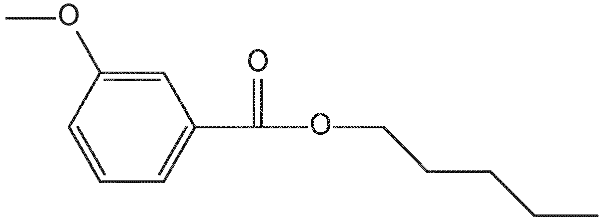
- 3-Methoxybenzoesäure-n-hexylester 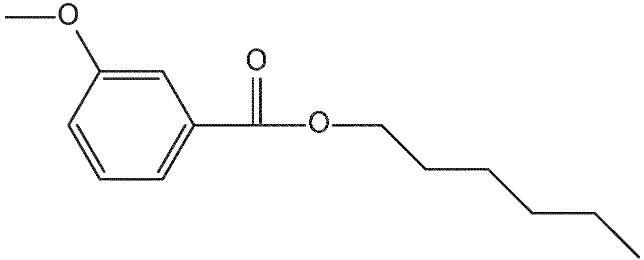
- 4-Methoxybenzoesäure 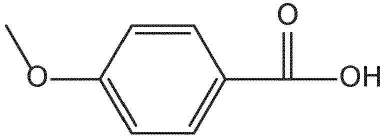
- 4-Methoxybenzoesäure- methylester 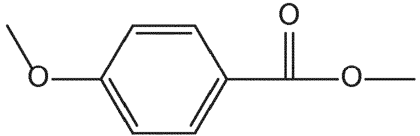
- 4-Methoxybenzoesäure-ethylester 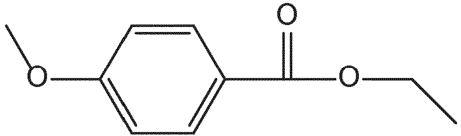
- 4-Methoxybenzoesäure- n-propylester 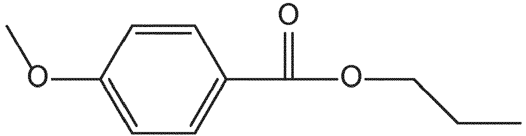
- 4-Methoxybenzoesäure-isopropylester 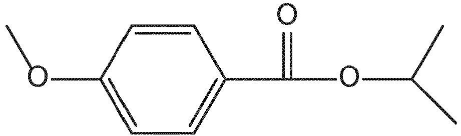
- 4-Methoxybenzoesäure-n-butylester 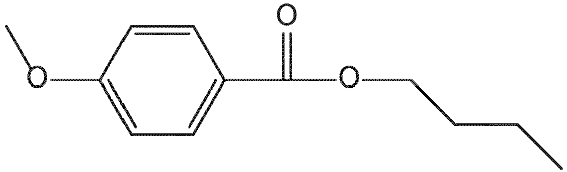
- 4-Methoxybenzoesäure-n-pentylester und/oder 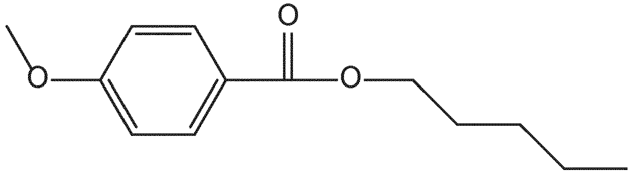
- 4-Methoxybenzoesäure-n-hexylester.

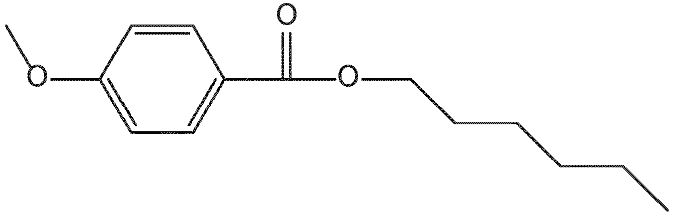

Im Rahmen einer ganz besonders bevorzugten Ausführungsform ist ein erfindungsgemäßes Verfahren dadurch gekennzeichnet, dass die zweite Zusammensetzung (B) eine oder mehrere aromatische Verbindungen (B2) der Formel (AR-I) enthält, die ausgewählt sind aus der Liste aus Thymol (2-Isopropyl-5-methyl-phenol), Benzylalkohol, Benzoesäure-n-pentylester, 4-Hydroxybenzoesäure-methylester, 4-Hydroxybenzoesäure-ethylester, 4-Hydroxybenzoesäure-n-propylester, Benzoesäure, Benzoesäuremethylester, Benzoesäure-ethylester, Benzoesäure-n-propylester, Benzoesäure-isopropylester, Benzoesäure-n-butylester, Benzoesäure-n-hexylester, 2-Hydroxybenzoesäure, 2-Hydroxybenzoesäure- methylester, 2-Hydroxybenzoesäure- ethylester, 2-Hydroxybenzoesäure- n-propylester, 2-Hydroxybenzoesäure-isopropylester, 2-Hydroxybenzoesäure-n-butylester, 2-Hydroxybenzoesäure-n-pentylester, 2-Hydroxybenzoesäure-n-hexylester, 3-Hydroxybenzoesäure, 3-Hydroxybenzoesäure- methylester, 3-Hydroxybenzoesäure- ethylester, 3-Hydroxybenzoesäure- n-propylester, 3-Hydroxybenzoesäure-isopropylester, 3-Hydroxybenzoesäure-n-butylester, 3-Hydroxybenzoesäure-n-pentylester, 3-Hydroxybenzoesäure-n-hexylester, 4-Hydroxybenzoesäure, 4-Hydroxybenzoesäure-isopropylester, 4-Hydroxybenzoesäure-n-butylester, 4-Hydroxybenzoesäure-n-pentylester, 4-Hydroxybenzoesäure-n-hexylester, 2-Methoxybenzoesäure, 2-Methoxybenzoesäure- methylester, 2-Methoxybenzoesäure- ethylester, 2-Methoxybenzoesäure- n-propylester, 2-Methoxybenzoesäure-isopropylester, 2-Methoxybenzoesäure-n-butylester, 2-Methoxybenzoesäure-n-pentylester, 2-Methoxybenzoesäure-n-hexylester, 3-Methoxybenzoesäure, 3-Methoxybenzoesäure- methylester, 3-Methoxybenzoesäure- ethylester, 3-Methoxybenzoesäure- n-propylester, 3-Methoxybenzoesäure-isopropylester, 3-Methoxybenzoesäure-n-butylester, 3-Methoxybenzoesäure-n-pentylester, 3-Methoxybenzoesäure-n-hexylester, 4-Methoxybenzoesäure, 4-Methoxybenzoesäure- methylester, 4-Methoxybenzoesäure-ethylester, 4-Methoxybenzoesäure- n-propylester, 4-Methoxybenzoesäure-isopropylester, 4-Methoxybenzoesäure-n-butylester, 4-Methoxybenzoesäure-n-pentylester und/oder 4-Methoxybenzoesäure-n-hexylester.

Im Rahmen einer explizit ganz besonders bevorzugten Ausführungsform ist ein erfindungsgemäßes Verfahren dadurch gekennzeichnet, dass die zweite Zusammensetzung (B) eine oder mehrere aromatische Verbindungen (B2) der Formel (AR-I) enthält, die ausgewählt sind aus der Liste aus Thymol (2-Isopropyl-5-methyl-phenol), Benzylalkohol, Benzoesäure-n-pentylester, 4-Hydroxybenzoesäure-methylester, 4-Hydroxybenzoesäure-ethylester, 4-Hydroxybenzoesäure-n-propylester und/oder Benzoesäure.

Alle zuvor beschriebenen Aromatische Verbindungen (B2) der Formel (AR-I) sind bei verschiedenen Chemikalien-Lieferanten wie beispielsweise Aldrich oder Fluka kommerziell erhältlich.

Durch Wahl der geeigneten Einsatzmengen an aromatische nVerbindungenn (B2) der Formel (AR-I) kann die Geschwindigkeit der von den C₁-C₆-Alkoxy-Silanen ausgehenden Filmbildung besonders stark mitbestimmt werden. Aus diesem Grund hat es sich als ganz besonders bevorzugt erwiesen, ein oder mehrere Aromatische Verbindungen (B2) in ganz bestimmten Mengenbereichen einzusetzen.

Es ist ganz besonders bevorzugt, wenn die zweite Zusammensetzung (B) - bezogen auf das Gesamtgewicht der Zusammensetzung (B) - ein oder mehrere aromatische Verbindungen (B2) der Formel (AR-I) in einer Gesamtmenge von 0,1 bis 35,0 Gew.-%, bevorzugt von 0,3 bis 15,0 Gew.-%, weiter bevorzugt von 0,5 bis 7,5 Gew.-% und ganz besonders bevorzugt von 1,0 bis 5,0 Gew.-% enthält.

Im Rahmen einer weiteren ganz besonders bevorzugten Ausführungsform ist ein erfindungsgemäßes Verfahren dadurch gekennzeichnet, dass die zweite Zusammensetzung (B) - bezogen auf das Gesamtgewicht der Zusammensetzung (B) - ein oder mehrere aromatische Verbindungen (B2) der Formel (AR-I) in einer Gesamtmenge von 0,1 bis 35,0 Gew.-%, bevorzugt von 0,3 bis 15,0 Gew.-%, weiter bevorzugt von 0,5 bis 7,5 Gew.-% und ganz besonders bevorzugt von 1,0 bis 5,0 Gew.-% enthält.

### Weitere kosmetische Inhaltsstoffe in der Zusammensetzung (B)

Die Zusammensetzung (B) kann zusätzlich auch noch einen oder mehrere weitere kosmetische Inhaltsstoffe enthalten.

Bei den fakultativ in der Zusammensetzung (B) einsatzbaren kosmetischen Inhaltsstoffen kann es sich um alle geeigneten Bestandteile handeln, um dem Mittel weitere positive Eigenschaften zu verleihen. Beispielsweise können in der Zusammensetzung (A) ein Lösungsmittel, ein verdickendes oder filmbildendes Polymer, eine oberflächenaktive Verbindung aus der Gruppe der nichtionischen, kationischen, anionischen oder zwitterionischen/amphoteren Tenside, der farbgebenden Verbindungen aus der Gruppe der Pigmente, der direktziehenden Farbstoffe, der Oxidationsfarbstoffvorprodukte, der Fettkomponenten aus der Gruppe der C₈-C₃₀-Fettalkohole, der Kohlenwasserstoffverbindungen, Fettsäureester, der zur Gruppe der pH-Regulatoren zugehörigen Säuren und Basen, der der Parfüme, der Konservierungsmittel, der Pflanzenextrakte und der Proteinhydrolysate enthalten sein.

Handelt es sich bei dem erfindungsgemäßen Verfahren um ein Verfahren zur Färbung von keratinischem Material, so kann die Zusammsentzung (B) ganz besonders bevorzugt mindestens eine färbende Verbindung aus der Gruppe der Pigmente und/oder der direktziehenden Farbstoffe enthalten.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen.

### pH-Werte der Zusammensetzungen im Verfahren

In weiteren Versuchen hat sich herausgestellt, dass die pH-Werte der Zusammensetzungen (A) und/oder (B) einen Einfluss auf die bei der Anwendung ablaufenden zuvor beschriebenen Hydrolyse- bzw. Kondensationsreaktionen nehmen können. Hierbei wurde gefunden, dass insbesondere alkalische pH-Werte die Kondensation auf der Stufe der Oligomere anhalten. Je saurer das Reaktionsgemisch ist, desto stärker scheint die Kondensation abzulaufen und desto höher ist das Molekulargewicht der bei der Kondensation entstehenden Silan-Kondensate. Aus diesem Grund ist es bevorzugt, dass die Zusammensetzungen (A) und/oder (B) einen pH-Wert von 7,0 bis 12,0, bevorzugt von 7,5 bis 11,5, weiter bevorzugt von 8,5 bis 11,0 und ganz besonders bevorzugt von 9,0 bis 11,0 besitzen.

Der Wassergehalt der Zusammensetzung (A) liegt bei maximal 10,0 Gew.-% und wird bevorzugt noch geringer eingestellt. Im Rahmen einiger Ausführungsformen kann auch der Wassergehalt der Zusammensetzung (B) gering gewählt sein. Insbesondere bei Zusammensetzungen mit sehr geringem Wassergehalt kann sich die Messung des pH-Wertes mit den üblichen aus dem Stand der Technik bekannten Methoden (pH-Wert Messung mittels Glaselektroden über Einstabmessketten oder aber über pH-Indikator-Papier) als schwierig erweisen. Aus diesem Grund handelt es sich bei den erfindungsgemäßen pH-Werten um die Werte, die nach Vermischen oder Verdünnung der Zubereitung im Gewichtsverhältnis 1:1 mit destilliertem Wasser erhalten wurden.

Der entsprechende pH-Wert wird dementsprechend gemessen, nachdem beispielsweise 50 g der erfindungsgemäßen Zusammensetzung mit 50 g destilliertem Wasser vermischt wurden.

In einer weiteren ganz besonders bevorzugten Ausführungsform ist ein erfindungsgemäßes Verfahren, dadurch gekennzeichnet, dass die Zusammensetzung (A) und/oder (B) nach der Verdünnung mit destilliertem Wasser im Gewichtsverhältnis 1:1 einen pH-Wert von 7,0 bis 11,5, weiter bevorzugt von 8,5 bis 11,0 und ganz besonders bevorzugt von 9,0 bis 11,0 besitzt.

Zur Einstellung dieses alkalischen pH-Wertes kann es erforderlich werden, der Reaktionsmischung ein Alkalisierungsmittel und/oder Acidifizierungsmittel zuzusetzen. Bei den pH-Werten im Sinne der vorliegenden Erfindung handelt es sich um pH-Werte, die bei einer Temperatur von 22°C gemessen wurden.

Als Alkalisierungsmittel können beispielsweise Ammoniak, Alkanolamine und/oder basische Aminosäuren eingesetzt werden.

Alkanolamine können ausgewählt werden aus primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methyl-propan-2-ol, 3-Aminopropan-1,2-diol, 2-Amino-2-methylpropan-1,3-diol.

Als Aminosäure im Sinne der Erfindung gilt eine organische Verbindung, die in ihrer Struktur mindestens eine protonierbare Aminogruppe und mindestens eine -COOH- oder eine -SO₃H-Gruppe enthält. Bevorzugte Aminosäuren sind Aminocarbonsäuren, insbesondere α-(alpha)-Aminocarbonsäuren und ω-Aminocarbonsäuren, wobei α-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus können auch anorganische Alkalisierungsmittel eingesetzt werden. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Neben den zuvor beschriebenen Alkalisierungsmitteln sind dem Fachmann zur Feineinstellung des pH-Wertes gängige Acidifizierungsmittel geläufig. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

### Anwendung der Zusammensetzungen (A) und (B)

Das erfindungsgemäße Verfahren umfass die Anwendung derr beiden Zusammensetzungen (A) und (B) auf dem keratinischen Material. Wesentlich für das Verfahren ist, dass die Zusammensetzungen (A) und (B) auf dem keratinischen Material miteinander in Kontakt kommen. Wie bereits zuvor beschrieben entsteht dieser Kontakt durch vorheriges Abmischen von (A) und (B).

Die zu dieser Erfindung führenden Arbeiten haben gezeigt, dass die wasserhaltige Zusammensetzung (B) mit den aromatischen Verbindungen (B2) insbesondere dann optimalen Einfluss auf den wasserarmen Silan-Blend (d.h. auf die Zusammensetzung (A)) nehmen kann, wenn die Zusammensetzungen (A) und (B) vor der Anwendung miteinander vermischt wurden.

Dieses Vermischen kann beispielsweise durch Verrrühren oder Verschütteln erfolgen. Ganz gesonders vorteilhaft ist es, die beiden Zusammensetzungen (A) und (B) getrennt in zwei Containern zu konfektionieren, und vor der Anwendung dann die gesamte Menge der Zusammensetzung (A) aus ihrem Container in den Container zu überführen, in dem sich die zweite Zusammsensetzung (B) befindet.

Das erfindungsgemäße Verfahren ist dadurch gekennzeichnet, dass auf dem keratinischen Material eine Zusammensetzung angewendet wird, die unmittelbar vor der Anwendung durch Vermischen der ersten Zusammensetzung (A) und der zweiten Zusammensetzung (B) hergestellt wurde.

Die beiden Zusammensetzungen (A) und (B) können in unterschiedlichen Mengenverhältnissen miteinander vermischt werden.

Besonders bevorzugt wird die Zusammensetzung (A) in Form eine relativ hoch konzentrierten, wasserarmen Silan-Blends eingesetzt, der durch Vermischen mit der Zusammensetzung (B) quasi verdünnt wird. Aus diesem Grund ist es ganz besonders bevorzugt, die Zusammensetzung (A) mit einem Gewichtsüberschuss an Zusammensetzung (B) zu vermischen. So kann beispielsweise 1 Gewichtsanteil (A) mit 20 Gewichtsanteilen (B) vermischt werden, oder 1 Gewichtsanteil (A) wird mit 10 Gewichtsanteilen (B) vermischt, oder aber 1 Gewichtsanteil (A) wird mit 5 Gewichtsanteilen (B) vermsicht.

Im Rahmen einer ganz besonders bevorzugten Ausführungsform ist ein erfindungsgemäßes Verfahren dadurch gekennzeichnet, dass auf dem keratinischen Material eine Zusammensetzung angewendet wird, die unmittelbar vor der Anwendung durch Vermischen der ersten Zusammensetzung (A) und der zweiten Zusammensetzung (B) in einem Mengenverhältnis (A)/(B) von 1:5 bis 1:20 hergestellt wurde.

Prinzipiell ist es aber auch möglich, die Zusammensetzung (A) im Verhältnis zur Zusammensetzung (B) in einem Gewichtsüberschuss einzusetzen. So können auch beipsielsweise 20 Gewichtsanteile (A) mit 1 Gewichtsanteil (B) vermischt werden, oder 10 Gewichtsanteile (A) werden mit 1 Gewichtsanteil (B) vermischt, oder aber 5 Gewichtsanteile (A) werden mit 1 Gewichtsanteil (B) vermischt.

Im Rahmen einer Ausführungsform können nur die beiden Zusammensetzungen (A) und (B) angewendet werden. Insbesondere bei Anwendung des erfindungsgemäßen Verfahrens zum Färben von keratinischem Material kann es auch ganz besonders bevorzugt sein, wenn auf dem Keratinmaterial nicht nur die beiden Zusammensetzungen (A) und (B), sondern weiterhin noch mindestens eine dritte Zusammensetzung (C) angewendet werden.

In einem Verfahren zur Färbung von keratinischem Material kann es sich bei der dritten Zusammensetzung (C ) beispielweise um eine Zusammensetzung handeln, die mindestens eine farbgebende Verbindung aus der Gruppe der Pigmente und/oder der direktziehenden Farbstoffe enthält.

Im Rahmen einer weiteren Ausführungsform ganz besonders bevorzugt ist ein erfindungsgemäßes Verfahren, bei dem auf dem keratinischen Material angewendet wird
- eine dritte Zusammensetzung (C), die enthält
   mindestens eine farbgebende Verbindung as der Gruppe der Pigmente und/oder der direktziehenden Farbstoffe.

Bei Anwendung der drei Zusammensetzungen (A), (B) und (C) sind verschiedene Ausführungsformen erfindungsgemäß.

Im Rahmen einer Ausführungsform ist es ganz besonders bevorzugt, vor der Anwendung eine Abmischung aus den drei Zusammsensetzungen (A), (B) und (C) herzustellen und dann dieses Gemisch dann auf dem Keratinmaterial anzuwenden.

Im Rahmen einer ganz besonders bevorzugten Ausführungsform ist ein erfindungsgemäßes Verfahren dadurch gekennzeichnet, dass auf dem keratinischen Material eine Zusammensetzung angewendet wird, die unmittelbar vor der Anwendung durch Vermischen der ersten Zusammensetzung (A) mit der zweiten Zusammensetzung (B) und einer dritten Zusammensetzung (C) erhalten wurde,
wobei die dritte Zusammensetzung (C) mindestens eine farbgebende Verbindung aus der Gruppe der Pigmente und/oder der direktziehenden Farbstoffe enthält.

Bei Färbung des Keratinmaterials kann es ebenfalls ganz besonders bevorzugt sein, unmittelbar vor der Anwendung durch Vermischen der ersten Zusammensetzung (A) und der zweiten Zusammensetzung (B) eine Mischung herzustellen und diese Mischung aus (A) und (B) auf dem Keratinmaterial anzuwenden. Die dritte Zusammensetzung (C) mit den farbgebenden Verbindungen kann dann im Anschluss daran auf das Keratinmaterial gegeben werden.

Im Rahmen einer ganz besonders bevorzugten Ausführungsform ist ein erfindungsgemäßes Verfahren dadurch gekennzeichnet, dass auf dem keratinischen Material eine Zusammensetzung angewendet wird, die unmittelbar vor der Anwendung durch Vermischen der ersten Zusammensetzung (A) mit der zweiten Zusammensetzung (B) erhalten wurde, und im Anschluss daran die die Zusammensetzung (C) auf dem keratinischen Material angewendet wird.

Mit anderen Worten ist ein ganz besonders bevorzugtes erfindungsgemäßes Verfahren dadurch gekennzeichnet, dass in einem ersten Schritt auf dem keratinischen Material eine Zusammensetzung angewendet wird, die unmittelbar vor der Anwendung durch Vermischen der ersten Zusammensetzung (A) und der zweiten Zusammensetzung (B) hergestellt wurde, und in einem zweiten Schritt auf dem keratinischen Material die dritte Zusammensetzung (C) angewendet wird.

Zusätzlich zu den Zusammensetzungen (A) und (B) - bzw. (A), (B) und (C) - kann im erfindungsgemäßen Verfahren auch noch eine vierte Zusammensetzung (D) auf dem Keratinmaterial appliziert werden. Die Anwendung der vierten Zusammensetzung (D) erfolgt besonders bevorzugt in einem Färbevefahren, um die zuvor erhaltenen Färbungen nochmals zu versiegeln. Für diese Versiegelung kann die Zusammensetzug (D) beispeilsweise mindestens ein filmbildendes Polymer enthalten.

Mit anderen Worten weiterhin ganz besonders bevorzugt ist ein erfindungsgemäßes Verfahren bei dem auf dem keratinischen Material angewendet wird
- eine vierte Zusammensetzung (D), die enthält
   mindestens eine filmbildendes Polymer.

### Farbgebende Verbindungen

Bei Anwendung der über das erfindungsgemäße Verfahren hergestellten Mittel in einem Färbeverfahren können eine oder mehrere farbgebende Verbindungen zum Einsatz kommen.

Insbesondere die Zubereitung (B) und/oder die fakultativ enthaltende Zubereitung (C) können zusätzlich mindestens eine farbgebende Verbindung enthalten.

Das oder die farbgebenden Verbindungen können vorzugsweise ausgewählt werden aus der Pigmente, der direktziehenden Farbstoffe, der Oxidationsfarbstoffe, der photochromen Farbstoffe und der thermochromen Farbstoffe, insbesondere bevorzugt aus Pigmenten und/oder direktziehenden Farbstoffen.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist en erfindungsgemäßes Mittel dadurch gekennzeichnet, dass es mindestens eine farbgebende Verbindung aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte farbgebende Verbindungen aus der Gruppe der Pigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Im Rahmen einer ganz besonders bevorzugten Ausführungsform ist ein erfindungsgemäßes Verfahren dadurch gekennzeichnet, dass die Zusammensetzung (B) und/oder die Zusammensetzung (C) mindestens eine farbgebende Verbindung aus der Gruppe der anorganischen Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Mittel dadurch gekennzeichnet, dass es (b) mindestens eine farbgebende Verbindung aus der Gruppe der Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbgebenden Verbindungen auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Mittel dadurch gekennzeichnet, dass es (b) mindestens eine farbgebende Verbindung enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} von der Firma Eckart Cosmetic Colors und Sunshine^{®} von der Firma Sunstar erhältlich.

Ganz besonders bevorzugte Farbpigmente mit der Handelsbezeichnung Colorona^{®} sind beispielsweise:
Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491(Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona^{®} sind beispielsweise:
Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.
Zudem sind besonders bevorzugte Farbpigmente mit der Handelsbezeichnung Unipure^{®} beispielsweise:
   Unipure Red LC 381 EM, Sensient CI 77491 (Iron Oxides), Silica
   Unipure Black LC 989 EM, Sensient, CI 77499 (Iron Oxides), Silica
   Unipure Yellow LC 182 EM, Sensient, CI 77492 (Iron Oxides), Silica

Im Rahmen einer weiteren Ausführungsform kann das erfindungsgemäße Mittel bzw. die erfindungsgemäße Zubereitung auch ein oder mehrere farbgebende Verbindungen aus der Gruppe der organischen Pigmente enthalten

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Verfahren dadurch gekennzeichnet, dass dass die Zusammensetzung (B) und/oder die Zusammensetzung (C) mindestens eine farbgebende Verbindung aus der Gruppe der organischen Pigmente enthält, die ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem erfindungsgemäßen Mitteln besonders bevorzugt. Weiterhin ist es bevorzugt, wenn die eingesetzten Pigmente eine bestimmte Teilchengröße aufweisen. Diese Teilchengröße führt einerseits zu einer gleichmäßigen Verteilung der Pigmente in dem gebildeten Polymerfilm und vermeidet andererseits ein raues Haar- oder Hautgefühl nach dem Auftragen des kosmetischen Mittels. Es ist daher erfindungsgemäß vorteilhaft, wenn das mindestens eine Pigment eine mittlere Teilchengröße D₅₀ von 1,0 bis 50 µm, vorzugsweise von 5,0 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

Das oder die Pigmente können in einer Menge von 0,001 bis 20 Gew.-%, insbesondere von 0,05 bis 5 Gew.-%, jeweils bezogen auf das Gesamtgewicht des erfindungsgemäßen Mittels bzw. der Zubereitung, eingesetzt werden.

Als farbgebende Verbindungen können die erfindungsgemäßen Mittel auch einen oder mehrere direktziehende Farbstoffe enthalten. Bei direktziehende Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1,0 g/L. Besonders bevorzugt besitzen die direktziehenden Farbstoffe im Sinne der vorliegenden Erfindung eine Löslichkeit in Wasser (760 mmHg) bei 25 °C von mehr als 1,5 g/L.

Direktziehende Farbstoffe können in anionische, kationische und nichtionische direktziehende Farbstoffe unterteilt werden.

In einer weiteren bevorzugten Ausführungsform ist ein erfindungsgemäßes Mittel dadurch gekennzeichnet, dass es als farbgebende Verbindung mindestens einen anionischen, kationischen und/oder nichtionischen direktziehenden Farbstoff enthält.

In einer weiteren bevorzugten Ausführungsform ist ein erfindungsgemäßes Verfahren dadurch gekennzeichnet, dass die Zusammensetzung (B) und/oder die Zusammensetzung (C) mindestens eine farbgebende Verbindung aus der Gruppe der anionischen, nichtionischen, und/oder kationischen direktziehenden Farbstoffe enthält.

Geeignete kationische direktziehende Farbstoffe sind beispielsweise Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76

Als nichtionische direktziehende Farbstoffe können beispielsweise nichtionische Nitro- und Chinonfarbstoffe und neutrale Azofarbstoffe eingesetzt werden. Geeigente nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung (-SO₃H) besitzen. In Abhängigkeit vom pH-Wert liegen die protonierten Formen (-COOH, -SO₃H) der Carbonsäure- bzw. Sulfonsäuregruppierungen mit ihren deprotonierten Formen (-COO⁻, -SO₃⁻ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Erfindungsgemäße Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1,0 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Als besonders gut geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, lodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intenstität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1,0 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriuimsalze von Mono- und Sisulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatirumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 GEw.-%.

Acid Red 33 ist das Diantriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1 ,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

Weiterhin können auch thermochrome Farbstoffe eingesetzt werden. Thermochromie beinhaltet die Eigenschaft eines Materials, in Abhängigkeit der Temperatur reversibel oder irreversibel seine Farbe zu ändern. Dies kann sowohl durch Änderung der Intensität und/oder des Wellenlängenmaximums erfolgen.

Schließlich ist es auch möglich, photochrome Farbstoffe einzusetzen. Photochromie beinhaltet die Eigenschaft eines Materials, in Abhängigkeit der Bestrahlung mit Licht, insbesondere UV-Licht, reversibel oder irreversibel seine Farbe zu ändern. Dies kann sowohl durch Änderung der Intensität und/oder des Wellenlängenmaximums erfolgen.

### Filmbildende Polymere

Die zuvor beschriebenen Zubereitungen, insbesondere die Zubereitungen (B), (C) und (D), ganz besonders bevorzugt die Zubereitung (D), können mindestens ein filmbildendes Polymer enthalten.

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen. Bei den Polymeren der vorliegenden Erfindung kann es sich um synthetisch hergestellte Polymere handeln, die durch Polymerisation eines Monomertyps oder durch Polymerisation verschiedener, strukturell voneinander unterschiedlicher Monomertypen hergestellt werden. Wird das Polymer durch Polymerisation eines Monomertyps hergestellt, spricht man von Homo-Polymeren. Werden strukturell unterschiedliche Monomertypen in der Polymerisation eingesetzt, wird das resultierende Polymer als Copolymer bezeichnet.

Das maximale Molekulargewicht des Polymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des filmbildenden, hydrophoben Polymers (c) nicht mehr als 10⁷ g/mol, bevorzugt nicht mehr als 10⁶ g/mol und besonders bevorzugt nicht mehr als 10⁵ g/mol beträgt.

Unter einem filmbildenden Polymer wird im Sinne der Erfindung ein Polymer verstanden, welches in der Lage ist, auf einem Substat, beispielsweise auf einem keratinischen Material oder einer keratinischen Faser, einen Film auszubilden. Die Ausbildung eines Films kann beispielsweise durch Betrachtung des mit dem Polymer behandelten Keratinmaterials unter einem Mikroskop nachgewiesen werden.

Die filmbildenden Polymere können hydrophil oder hydrophob sein.

Im Rahmen einer ersten Ausführungsform kann es bevorzugt sein, in der Zubereitung (B), (C) und/oder (D), ganz besonders in der Zubereitung (D), mindestens ein hydrophobes, filmbildendes Polymer einzusetzen.

Unter einem hydrophoben Polymer wird ein Polymer verstanden, dass eine Löslichkeit in Wasser bei 25 °C (760 mmHg) von weniger als 1 Gew.-% besitzt.

Die Wasserlöslichkeit des filmbildenden, hydrophoben Polymers kann beispielsweise auf dem folgenden Weg bestimmt werden. 1,0 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Sofern sich die Polymer-Wasser-Mischung aufgrund einer hohen Trübung des Gemisches nicht visuell beurteilten lässt, wird die Mischung filtriert. Bleibt auf dem Filterpapier ein Anteil an ungelöstem Polymer zurück, dann liegt die Löslichkeit des Polymers bei weniger als 1 Gew.-%.

Hier können insbesondere die Polymere des Acrylsäure-Typs, die Polyurethane, die Polyester, die Polyamide, die Polyharnstoffe, die Cellulose-Polymere, die Nitro-Cellulose-Polymere, die Silikon-Polymere, die Polymere des Acrylamid-Typs und die Polyisoprene genannt werden.

Besonders gut geeignete filmbildende, hydrophobe Polymere sind beispielsweise Polymere aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

In einer weiteren bevorzugten Ausführungsform ist ein erfindungsgemäßes Mittel dadurch gekennzeichnet, dass es mindestens ein filmbildendes, hydrophobes Polymer (c) enthält, das ausgewählt ist aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Zur Lösung der erfindungsgemäßen Aufgabenstellung haben sich insbesondere die filmbildenden hydrophoben Polymere als gut geeignet erwiesen, die aus der Gruppe der synthetischen Polymere, der durch radikalische Polymerisation erhältlichen Polymere oder der natürlichen Polymere ausgewählt werden.

Weitere besonders gut geeignete filmbildende hydrophobe Polymere können ausgewählt werden aus den Homopolymere oder Copolymeren von Olefinen, wie beispielsweise Cycloolefinen, Butadien, Isopren oder Styren, Vinylethern, Vinylamiden, den Estern oder Amiden von (Meth)Acrylsäure mit mindestens einer C₁-C₂₀-Alkylgruppe, einer Arylgruppe oder einer C₂-C₁₀-Hydroxyalkylgruppe.

Weitere filmbildende hydrophobe Polymere können ausgewählt sein aus den Homo- oder Copolymeren von Isooctyl-(meth)acrylat; Isononyl-(meth)acrylat; 2-Ethylhexyl-(meth)acrylat; Lauryl-(meth)acrylat); isopentyl-(meth)acrylat; n-Butyl-(meth)acrylat); Isobutyl-(meth)acrylat; Ethyl-(meth)acrylat; Methyl-(meth)acrylat; tert-Butyl-(meth)acrylat; Stearyl-(meth)acrylat; Hydroxyethyl-(meth)acrylat; 2-Hydroxypropyl-(methacrylat; 3-Hydroxypropyl-(meth)acrylat und/oder Gemischen hiervon.

Weitere filmbildende hydrophobe Polymere können ausgewählt sein aus den Homo- oder Copolymeren von (Meth)acrylamid; N-Alkyl-(meth)acrylamiden, insbesondere solchen mit C2-C18 Alkylgruppen, wie beispielsweise N-Ethyl-acrylamid, N-tert-butyl-acrylamid, le N-Octyl-crylamid; N-Di(C1-C4)alkyl-(meth)acrylamid.

Weitere bevorzugte anionische Copolymere sind beispielsweise Copolymere aus Acrylsäure, Methacrylsäure oder deren C₁-C₆-Alkylestern, wie sie unter der INCI-Deklaration Acrylates Copolymere vertrieben werden. Ein geeignetes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁-C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20.

Auf dem Markt befindliche ganz besonders bevorzugte Polymere sind beispielsweise Aculyne 22 (Acrylates/Steareth-20 Me-thacrylate Copolymer), Aculyne 28 (Acrylates/Beheneth-25 Methacrylate Copolymer), Structure 2001^{®} (Acryla-tes/Steareth-20 Itaconate Copolymer), Structure 3001^{®} (Acrylates/Ceteth-20 Itaconate Copolymer), Structure Plus^{®} (Acrylates/Aminoacrylates C10-30 Alkyl PEG-20 Itaconate Copolymer), Carbopol^{®} 1342, 1382, Ultrez 20, Ultrez 21 (Acrylates/C10-30 Alkyl Acrylate Crosspolymer), Synthalen W 2000^{®} (Acrylates/Palmeth-25 Acrylate Copolymer) oder das Rohme und Haas vertriebene Soltex OPT (Acrylates/C12-22 Alkyl methacrylate Copolymer).

Als geeignete Polymere auf der Basis von Vinyl-Monomeren können beispielsweise genannt werden die Homo- und Copolymere des N-Vinylpyrrolidons, des Vinylcaprolactams, des Vinyl-(C1-C6-)alkyl-Pyrrols, des Vinyl-oxazols, des Vinyl-thiazols, des Vinylpyrimidins, des Vinylimidazols.

Weiterhin ganz besonders gut geeignet sind die Copolymere Octylacrylamid/acrylates/ butylaminoethyl-methacrylate copolymer, wie es beispielsweise unter den Handelsnamen AMPHOMER^{®} ou LOVOCRYL^{®} 47 von NATIONAL STARCH kommerziell vertrieben wird, oder auch die Copolymere von Acrylates/Octylacrylamide die unter den Handelsnamen DERMACRYL^{®} LT und DERMACRYL^{®} 79 von NATIONAL STARCH vertrieben werden.

Als geeignete Polymere auf der Basis von Olefinen können beispielsweise genannt werden die Homo- und Copolymere des Ethylens, des Propylens, des Butens, des Isoprens und des Butadiens.

Im Rahmen einer weiteren Ausführungsform können als filmbildenden hydrophoben Polymere die Block-Copolymere eingesetzt werden, die mindestens einen Block aus Styren oder den Derivaten des Styrens umfassen. Bei diesen Block-Copolymeren kann es sich um Copolymere handeln, die neben einem Styren-Block einen oder mehrere weitere Blöcke enthalten, wie beispielsweise Styren/Ethylen, Styren/Ethylen/Butylen, Styen/Butylen, Styren/Isopren, Styren/Butadien. Entsprechende Polymere werden von der BASF unter dem Handelsnamen "Luvitol HSB" kommerziell vertrieben.

Es konnten auch dann intensive und waschechte Färbungen erhalten werden konnten, wenn die Zubereitung (B), (C) und/oder (D), ganz besonders in die Zubereitung (D), mindestens ein filmbildendes Polymer enthielt, das ausgewählt wurde aus der Gruppe der der Homopolymere und Copolymere von Acrylsäure, der Homopolymere und Copolymere von Methacrylsäure, der Homopolymere und Copolymere von Acrylsäure-Estern, der Homopolymere und Copolymere von Methacrylsäure-Estern, der Homopolymere und Copolymere von Acrylsäureamiden, der Homopolymere und Copolymere von Methacrylsäure-Amiden, der Homopolymere und Copolymere des Vinylpyrrolidons, der Homopolymere und Copolymere des Vinylalkohols, der Homopolymere und Copolymere des Vinylacetats, der Homopolymere und Copolymere des Ethylens, der Homopolymere und Copolymere des Propylens, der Homopolymere und Copolymere des Styrens, der Polyurethane, der Polyester und der Polyamide.

In einer weiteren bevorzugten Ausführungsform ist ein erfindungsgemäßes Verfahren dadurch gekennzeichnet, dass die Zubereitung (B), (C) und/oder (D), ganz besonders die Zubereitung (D), mindestens ein filmbildendes Polymer enthält, das ausgewählt ist aus der Gruppe der Homopolymere und Copolymere von Acrylsäure, der Homopolymere und Copolymere von Methacrylsäure, der Homopolymere und Copolymere von Acrylsäure-Estern, der Homopolymere und Copolymere von Methacrylsäure-Estern, der Homopolymere und Copolymere von Acrylsäureamiden, der Homopolymere und Copolymere von Methacrylsäure-Amiden, der Homopolymere und Copolymere des Vinylpyrrolidons, der Homopolymere und Copolymere des Vinylalkohols, der Homopolymere und Copolymere des Vinylacetats, der Homopolymere und Copolymere des Ethylens, der Homopolymere und Copolymere des Propylens, der Homopolymere und Copolymere des Styrens, der Polyurethane, der Polyester und der Polyamide.

Im Rahmen einer ersten Ausführungsform kann es bevorzugt sein, in der Zubereitung (B), (C) und/oder (D), ganz besonders in der Zubereitung (D), mindestens ein hydrophiles, filmbildendes Polymer einzusetzen.

Unter einem hydrophilen Polymer wird ein Polymer verstanden, dass eine Löslichkeit in Wasser bei 25 °C (760 mmHg) von mehr als 1 Gew.-%, bevorzugt von mehr als 2 Gew.-%, besitzt.

Die Wasserlöslichkeit des filmbildenden, hydrophilen Polymers kann beispielsweise auf dem folgenden Weg bestimmt werden. 1,0 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Ein vollständig gelöstes Polymer erscheint markoskopisch homogen. Sofern sich die Polymer-Wasser-Mischung aufgrund einer hohen Trübung des Gemisches nicht visuell beurteilten lässt, wird die Mischung filtriert. Bleibt auf dem Filterpapier kein ungelöstes Polymer zurück, dann liegt die Löslichkeit des Polymers bei mehr als 1 Gew.-%.

Als filmbildende, hydrophile Polymere können nichtionische, anionische und kationische Polymere eingesetzt werden.

Geeignete filmbildende, hydrophile Polymere können beispielsweise aus der Gruppe der Polyvinylpyrrolidon-(Co)Polymere, der Polyvinylalkohol-(Co)Polymere, der Vinylacetat-(Co)Polymere, der Carboxyvinyl-(Co)Polymere, der Acrylsäure-(Co)Polymere, der Methacrylsäure-(Co)Polymere, der natürlichen Gummen, der Polysaccharide und/oder der Acrylamid-(Co)Polymere ausgewählt werden.

Weiterhin ist es ganz besonders bevorzugt, als filmbildendes hydrophiles Polymer Polyvinylpyrrolidon (PVP) und/oder ein Vinylpyrrolidon-haltiges Copolymer einzusetzen.

In einer weiteren ganz besonders bevorzugten Ausführungsform ist ein erfindungsgemäßes Mittel dadurch gekennzeichnet, dass es (c) mindestens ein filmbildendes, hydrophiles Polymer enthält, das ausgewählt ist aus der Gruppe aus Polyvinylpyrrolidon (PVP) und den Copolymeren des Polyvinylpyrrolidons.

Es ist weiterhin bevorzugt, wenn das erfindungsgemäße Mittel als filmbildendes, hydrophiles Polymer Polyvinylpyrrolidon (PVP) enthält. Überraschenderweise war auch die Waschechtheit der Färbungen, die mit mit PVP-haltigen Mitteln (b9 erhalten werden konnten, sehr gut.

Besonders gut geeignete Polyvinylpyrrolidone sind beispielsweise unter der Bezeichnung Luviskol^{®} K von der BASF SE erhältlich, insbesondere Luviskole K 90 oder Luviskole K 85 der Firma BASF SE.

Als weiteres explizit ganz besonders gut geeignetes Polyvinylpyrrolidon (PVP) kann auch das Polymer PVP K30 eingesetzt werden, das von der Firma Ashland (ISP, POI Chemical) vertrieben wird. PVP K 30 ist ein in kaltem Wasser sehr gut lösliches Polyvinylpyrrolidon, welches die CAS-Nummer 9003-39-8 besitzt. Das Molgewicht von PVP K 30 liegt bei ca. 40000 g/mol.

Weitere ganz besonders gut geeignete Polyvinylpyrrolidone sind die unter den Handelsnamen LUVITEC K 17, LUVITEC K 30, LUVITEC K 60, LUVITEC K 80, LUVITEC K 85, LUVITEC K 90 und LUVITEC K 115 bekannten und von der BASF erhältlichen Substanzen.

Der Einsatz von filmbildenden hydrophilen Polymeren aus der Gruppe der Copolymere des Polyvinylpyrrolidons hat ebenfalls zu besonders guten und waschechten Farbergebnissen geführt.

Als besonders gut geeignete filmbildende, hydrophile Polymere können in diesem Zusammenhang Vinylpyrrolidon-Vinylester-Copolymere genannt werden, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind besonders bevorzugte nichtionische Polymere.

Von den Vinylpyrrolidon-haltigen Copolymeren werden ganz besonders bevorzugt ein Styrene/VP Copolymer und/oder ein Vinylpyrrolidon-Vinylacetat-Copolymer und/oder ein VP/DMAPA Acrylates Copolymer und/oder ein VP/Vinyl Caprolactam/DMAPA Acrylates Copolymer in den kosmetischen Zusammensetzungen eingesetzt.

Vinylpyrrolidon-Vinylacetat-Copolymere werden unter der Bezeichnung Luviskol^{®} VA von der BASF SE vertrieben. Ein VP/Vinyl Caprolactam/DMAPA Acrylates Copolymer wird beispielsweise unter dem Handelsnamen Aquaflex^{®} SF-40 von Ashland Inc. vertrieben. Ein VP/DMAPA Acrylates Copolymer wird beispielsweise unter der Bezeichnung Styleze CC-10 von Ashland vertrieben und ist ein höchst bevorzugtes Vinylpyrrolidon-haltiges Copolymer.

Als weitere geeignete Copolymere des Polyvinylpyrrolidons können auch die Copolymere genannt werden, die durch Umsetzung von N-Vinylpyrrolidon mit mindestens einem weiteren Monomer aus der Gruppe aus V-Vinylformamid, Vinylacetat, Ethylen, Propylen, Acrylamid, Vinylcaprolactam, Vinylcaprolacton und/oder Vinylalkohol erhalten werden.

In einer weiteren ganz besonders bevorzugten Ausführungsform ist ein erfindungsgemäßes Mittel dadurch gekennzeichnet, dass es mindestens ein filmbildendes, hydrophiles Polymer enthält, das ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/Ethylen-Copoylmeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren.

Ein weiteres geeigetes Copolymer des Vinylpyrrolidons ist das unter der INCI Bezeichnung Maltodextrin/VP Copolymer bekannte Polymer.

Weiterhin konnte intensiv gefärbtes Keratinmaterial, insbesondere Haare, mit sehr guten Waschechtheiten erhalten werden, wenn als filmbildendes, hydrophiles Polymer ein nichtionisches, filmbildendes, hydrophiles Polymer eingesetzt wurde.

Rahmen einer ersten Ausführungsform kann es bevorzugt sein, wenn die Zubereitung (B), (C) und/oder (D), ganz besonders die Zubereitung (D), mindestens ein nichtionisches, filmbildendes, hydrophiles Polymer entalten.

Unter einem nichtionischen Polymer wird erfindungsgemäß ein Polymer verstanden, das in einem protischen Lösemittel - wie beispielsweise Wasser - bei Standardbedingungen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Es sind die Mittel ganz besonders bevorzugt, die als nichtionisches, filmbildendes, hydrophiles Polymer mindestens ein Polymer enthalten, das ausgewählt ist aus der Gruppe aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymeren aus N-Vinylpyrrolidon und N-Vinylimidazol und Methacrylamid,
- Copolymeren aus N-Vinylpyrrolidon und N-Vinylimidazol und Acrylamid,
- Copolymeren aus N-Vinylpyrrolidon mit N,N-Di(C₁ bis C₄)-Alkylamino-(C₂ bis C₄)-alkylacrylamid.

Kommen Copolymere aus N-Vinylpyrrolidon und Vinylacetat zum Einsatz, ist es wiederum bevorzugt, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt. Geeignete Copolymerisate aus Vinylpyrrolidon und Vinylacetat sind beispielsweise unter dem Warenzeichen Luviskol^{®} VA 37, Luviskol^{®} VA 55, Luviskol^{®} VA 64 und Luviskol^{®} VA 73 von der Firme BASF SE erhältlich.

Ein weiteres besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Ein weiteres ganz besonders bevorzugtes nichtionisches, filmbildendes, hydrophiles Polymer st ein Copolymer aus N-Vinylpyrrolidon und N,N-Dimethylaminiopropylmethacrylamid,welches beispielsweise mit der INCI-Bezeichnung VP/DMAPA Acrylates Copolymer z. B. unter der Handelsbezeichnung Styleze^{®} CC 10 von der Firma ISP verkauft wird.

Ein kationisches erfindungsgemäßes Polymer ist das Copolymer aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle^{®} 300 (28-32 Gew.-% Aktivsubstanz in Ethanol-WasserGemisch, Molekulargewicht 350000) von der Firma ISP vertrieben wird.

Weitere geeignete filmbildende, hydrophile Polymere sind beispielsweise
- Vinylpyrrolidon-Vinylimidazoliummethochlorid-Copolymere, wie sie unter den Bezeichnungen Luviquat^{®} FC 370, FC 550 und der INCI-Bezeichnung Polyquaternium-16 sowie FC 905 und HM 552 angeboten werden,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Polyquaternium-11 ist das Reaktionsprodukt von Diethylsulfat mit einem Copolymer von Vinylpyrrolidon und Dimethylaminoethylmethacrylat. Geeignete Handelsprodukte sind beispielsweise unter den Bezeichnungen Dehyquart^{®} CC 11 und Luviquat^{®} PQ 11 PN von der BASF SE oder Gafquat 440, Gafquat 734, Gafquat 755 oder Gafquat 755N von Ashland Inc. erhältlich.

Polyquaternium-46 ist das Reaktionsprodukt von Vinylcaprolactam und Vinylpyrrolidon mit Methylvinylimidazoliummethosulfat und ist beispielsweise unter der Bezeichnung Luviquat^{®} Hold von der BASF SE erhältlich. Polyquaternium-46 wird bevorzugt in einer Menge von 1 bis 5 Gew.-% - bezogen auf das Gesamtgewicht der kosmetischen Zusammensetzung - eingesetzt. Es ganz besonders bevorzugt, dass Polyquaternium-46 in Kombination mit einer kationischen Guar-Verbindung eingesetzt wird. Es ist sogar höchst bevorzugt, dass Polyquaternium-46 in Kombination mit einer kationischen Guar-Verbindung und Polyquaternium-11 eingesetzt wird.

Als geeignete anionische filmbildende, hydrophile Polymere können zum Beispiel Acrylsäure-Polymere eingesetzt werden, die in unvernetzter oder vernetzter Form vorliegen können. Entsprechende Produkte werden beispielsweise unter den Handelsnamen Carbopol 980, 981, 954, 2984 and 5984 von der Firma Lubrizol oder auch unter den Namen Synthalen M and Synthalen K von der Firma 3V Sigma (The Sun Chemicals, Inter Harz) kommerziell vertrieben.

Beispiele für geeignete filmbildende, hydrophile Polymere aus der Gruppe der natürlichen Gums sind Xanthan Gum, Gellan Gum, Carob Gum .

Beispiele für geeignete filmbildende, hydrophile Polymere aus der Gruppe der Polysaccharide sind Hydroxyethylcellulose, Hydroxypropylcellulose, Ethylcellulose und Carboxymethyl-Cellulose.

Geeignete filmbildende, hydrophile Polymere aus der Gruppe der Acrylamde sind beispielsweise Polymere, welche ausgehend von Monomeren der (Methy)Acrylamido-C1-C4-alkyl-sulfonsäure bzw. der Salze hiervon hergestellt werden. Entsprechende Polymere können ausgewählt sein aus den Polymeren von Polyacrylamidomethansulfonsäure, Polyacrylamidoethansulfonsäure, Polyacrylamidopropansulfonsäure, Poly2-acrylamido-2-methylpropansulfonsäure, Poly-2-Methylacrylamido-2-methylpropansulfonsäure und/oder Poly-2-methylacrylamido-n-butansulfonsäure.

Bevorzugte Polymere der Poly(meth)arylamido-C1-C4-alkyl-sulfonsäuren sind vernetzt und zu mindestens 90 % neutralisiert. Diese Poylmere können vernetzt oder auch unvernetzt sein.

Vernetzte und ganz oder teilweise neutraliserte Polymere des Typs der Poly-2-acrylamido-2-methylpropansulfonsäuren sind unter den INCI-Namen "Ammonium Polyacrylamido-2-methyl-propanesulphonate" oder "Ammonium Polyacryldimethyltauramide" bekannt.

Ein weiteres bevorzugtes Polymer dieses Typs ist das der Firma Clamant unter dem Handelsnamen Hostacerin AMPS vertriebene vernetzte Poly-2-acrylamido-2methyl-propanesulphonsäure-Polymer, das teilweise mit Ammoniak neutralisiert ist.

In einer weiteren explizit ganz besonders bevorzugten Ausführungsform ist ein erfindungsgemäßes Verfahren dadurch gekennzeichnet, dass die Zubereitung (B), (C) und/oder (D), ganz besonders die Zubereitung (D), mindestens ein anionisches, filmbildendes, Polymer enthält.

In diesem Zusammenhang konnten die besten Ergebnisse erhalten werden, wenn die Zubereitung (B), (C) und/oder (D), ganz besonders die Zubereitung (D), mindestens ein filmbildendes Polymer enthält, das mindestens eine Struktureinheit der Formel (P-I) und mindestens eine Struktureinheit der Formel (P-II) umfasst 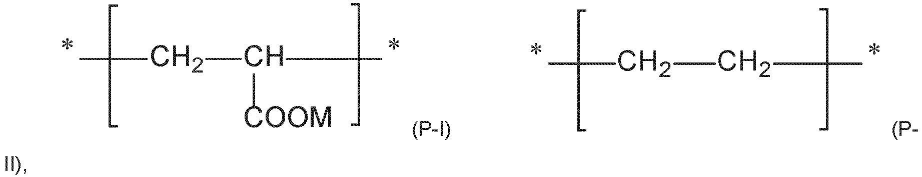 wobei
M für ein Wasserstoffatom oder für Ammonium (NH₄), Natrium, Kalium, ½ Magnesium oder ½ Calcium steht.

In einer weiteren bevorzugten Ausführungsform ist ein erfindungsgemäßes Verfahren dadurch gekennzeichnet, dass die Zubereitung (B), (C) und/oder (D), ganz besonders die Zubereitung (D), mindestens ein filmbildendes Polymer enthält, das mindestens eine Struktureinheit der Formel (P-I) und mindestens eine Struktureinheit der Formel (P-II) umfasst 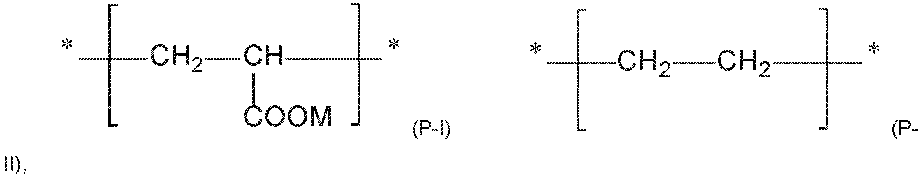 wobei
M für ein Wasserstoffatom oder für Ammonium (NH₄), Natrium, Kalium, ½ Magnesium oder ½ Calcium steht.

Wenn M für ein Wasserstoffatom steht, basiert die Struktureinheit der Formel (P-I) auf einer Acrylsäure-Einheit.

Wenn M für ein Ammonium-Gegenion steht, basiert die Struktureinheit der Formel (P-I) auf dem Ammoniumsalz der Acrylsäure.

Wenn M für ein Natrium-Gegenion steht, basiert die Struktureinheit der Formel (P-I) auf dem Natriumsalz der Acrylsäure.

Wenn M für ein Kalium-Gegenion steht, basiert die Struktureinheit der Formel (P-I) auf dem Kaliumsalz der Acrylsäure.

Wenn M für ein halbes Äquivalent eines Magnesium-Gegenions steht, basiert die Struktureinheit der Formel (P-I) auf dem Magnesiumsalz der Acrylsäure.

Wenn M für ein halbes Äquivalent eines Calcium-Gegenions steht, basiert die Struktureinheit der Formel (P-I) auf dem Calciumsalz der Acrylsäure.

Das oder die erfindungsgemäßen filmbildenden Polymere werden bevorzugt in bestimmten Mengenbereichen im erfindungsgemäßen Zubereitungen (B), (C) und/oder (D) eingesetzt. In diesem Zusammenhang hat es sich zur Lösung der erfindungsgemäßen Aufgabenstellung als besonders bevorzugt erwiesen, wenn die Zubereitung - jeweils bezogen auf ihr Gesamtgewicht- ein oder mehrere filmbildende Polymere in einer Gesamtmenge von 0,1 bis 18,0 Gew.-%, bevorzugt von 1,0 bis 16,0 Gew.-%, weiter bevorzugt von 5,0 bis 14,5 Gew.-% und ganz besonders bevorzugt von 8,0 bis 12,0 Gew.-% enthält.

In einer weiteren bevorzugten Ausführungsform ist ein erfindungsgemäßes Verfahren dadurch gekennzeichnet, dass die Zubereitung (B), (C) und/oder (D) - bezogen auf ihr jewiliges Gesamtgewicht- ein oder mehrere filmbildende Polymere in einer Gesamtmenge von 0,1 bis 18,0 Gew.-%, bevorzugt von 1,0 bis 16,0 Gew.-%, weiter bevorzugt von 5,0 bis 14,5 Gew.-% und ganz besonders bevorzugt von 8,0 bis 12,0 Gew.-% enthält.

### Beispiele

### 1. Herstellung des Silan-Blends (Zusammensetzung (A))

Ein Reaktor mit beheizbarer/kühlbarer Außenhülle und mit einem Fassungsvermögen von 10 Litern wurde mit 4,67 kg Methyltrimethoxysilan (34,283 mol) befüllt. Unter Rühren wurden dann 1,33 kg (3-Aminopropyl)triethoxysilan (6,008 mol) hinzugegeben. Dieses Gemisch wurde bei 30 °C gerührt. Im Anschluss daran wurden 670 ml destilliertes Wasser (37,18 mol) tropfenweise unter kräftigem Rühren hinzugegeben, wobei die Temperatur des Reaktionsgemisches unter externer Kühlung bei 30 °C gehalten wurde. Nach Beendigung der Wasserzugabe wurde für weitere 10 Minuten nachgerührt. Danach wurde ein Vakuum von 280 mbar angelegt und das Reaktionsgemisch auf eine Temperatur von 44 °C erwärmt. Sobald das Reaktionsgemisch die Temperatur von 44 °C erreicht hatte, wurden das bei der Reaktion freigesetzte Ethanol und Methanol über einen Zeitraum von 190 Minuten abdestilliert. Im Verlauf der Destillation wurde das Vakuum auf 200 mbar abgesenkt. Die abdestillierten Alkohole wurden in einer gekühlten Vorlage aufgefangen. Danach ließ man das Reaktionsgemisch auf Raumtemperatur abkühlen. Zu dem auf diese Weise erhaltenen Gemisch wurden dann unter Rühren 3,33 kg Hexamethyldisiloxan getropft. Es wurde 10 Minuten nachgerührt. Jeweils 100 ml des Silan-Blends wurden in eine Flasche mit 100 ml Fassungsvermögen und Schraubdeckelverschluss mit Dichtung abgefüllt. Nach dem Abfüllen wurden die Flaschen fest verschlossen. Der Wassergehalt betrug kleiner 2,0 Gew.-%.

### 2. Herstellung der Zusammensetzung (B)

Es wurden die folgenden Zusammensetzungen hergestellt (sofern nichts anderes angegebne ist, sind alle Angaben in Gew.-%).

### Zusammensetzung (B)

| | B-V1 Vergleich | B-E1 Erfindung | B-E2 Erfindung | B-E3 Erfindung |
|---|---|---|---|---|
| Hydroxyethylcellulose | 1,0 | 1,0 | 1,0 | 1,0 |
| Thymol ((2-Isopropyl-5-methyl-phenol) | --- | 5,0 | --- | --- |
| Carl Roth GmbH | | | | |
| Benzoesäure-n-pentylester | --- | --- | 5,0 | - |
| Sigma Aldrich | | | | |
| n-Pentylbenzoat | --- | --- | --- | 5,0 |
| abcr GmbH Product List | | | | |
| Wasser (dest.) | ad 100 | ad 100 | ad 100 | ad 100 |

### 3. Herstellung der Zusammensetzungen (C) und (D)

Es wurden die folgenden Zusammensetzungen hergestellt (sofern nichts anderes angegeben ist, sind alle Angaben in Gew.-%).

### Zusammensetzung (C)

| | Gew.-% |
|---|---|
| Lavanya Belmont | 35,0 |
| Phthalocyanin Blaupigment CI 74160 | |
| PEG-12 Dimethicone | ad 100 |

### Zusammensetzung (D)

| | Gew.-% |
|---|---|
| Ethylene/Sodium Acrylate Copolymer | 40,0 |
| 25%ige Lösung | |
| Wasser | ad 100 |

### 5. Anwendung

Die anwendungsbereite Zusammensetzung wurde jeweils durch Vermischen von 1,5 g der Zusammensetzung (A), 20,0 g der Zusammensetzung (B) und 1,5 g der Zusammensetzung (C) hergestellt. Die Zusammensetzungen (A), (B) und (C) wurden jeweils für 1 Minute verschüttelt, Dann wurde dieses anwendungsbereite Mittel jeweils auf zwei Haarsträhnen ausgefärbt.

Drei Minuten nach Beendigung des Verschüttelns wurde die anwendungsbereite Zusammensetzung auf eine erste Strähne (Strähne 1) appliziert, 1 min einwirken gelassen, und danach ausgespült. 10 Minuten nach Beendigung des Verschüttelns wurde die anwendungsbereite Zusammensetzung auf eine zweite Strähne (Strähne 2) appliziert, 1 min einwirken gelassen und danach ausgespült.

Im Anschluss daran wurde die Zusammensetzung (D) auf jede Haarstähne appliziert, für 1 Minute einwirken gelassen und danach ebenfalls mit Wasser ausgespült.

Die beiden gefärbten Strähnen wurden jeweils getrocknet und visuell unter einer Tageslichtlampe miteinander verglichen.

| | | | | |
|---|---|---|---|---|
| Schritt 1: | (A) + (B-V1) + (C) | (A) + (B-E1) + (C) | (A) + (B-E2) + (C) | (A) + (B-E3) + (C) |
| Schritt 2: | (D) | (D) | (D) | (D) |
| Farbunterschied | hoch | gering | gering | gering |

| | | | | |
|---|---|---|---|---|
| Farbunterschied = Farbunterschied zwischen Strähne 1 und Strähne 2 | | | | |

## Patentansprüche

1. Verfahren zum Behandeln von keratinischem Material, insbesondere menschlichen Haaren, bei dem auf dem keratinischen Material eine Zusammensetzung angewendet wird, die unmittelbar vor der Anwendung durch Vermischen einer ersten Zusammensetzung (A) und einer zweiten Zusammensetzung (B) hergestellt wurde, wobei
- die erste Zusammensetzung (A) - bezogen auf das Gesamtgewicht der Zusammensetzung (A)
- enthält
(A1) weniger als 10 Gew.-% Wasser und
(A2) mindestens ein organisches C₁-C₆-Alkoxy-Silan das ausgewählt ist aus der Gruppe aus
- (3-Aminopropyl)triethoxysilan
- (3-Aminopropyl)trimethoxysilan
- (2-Aminoethyl)triethoxysilan
- (2-Aminoethyl)trimethoxysilan
- (3-Dimethylaminopropyl)triethoxysilan
- (3-Dimethylaminopropyl)trimethoxysilan
- (2-Dimethylaminoethyl)triethoxysilan,
- (2-Dimethylaminoethyl)trimethoxysilan
und/oder deren Kondensationsprodukten, und
mindestens ein organisches C₁-C₆-Alkoxysilan, das ausgewählt ist aus der Gruppe aus
- Methyltrimethoxysilan
- Methyltriethoxysilan
- Ethyltrimethoxysilan
- Ethyltriethoxysilan
- Hexyltrimethoxysilan
- Hexyltriethoxysilan
- Octyltrimethoxysilan
- Octyltriethoxysilan
- Dodecyltrimethoxysilan,
- Dodecyltriethoxysilan,
und/oder deren Kondensationsprodukten, und
- die zweite Zusammensetzung (B) enthält
(B1) Wasser und
(B2) eine oder mehrere aromatische Verbindungen der Formel (AR-I) 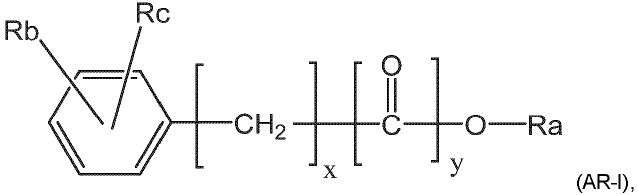 wobei
x für eine ganze Zahl von 0 bis 3 steht,
y für die Zahl 0 oder 1 seht,
Rₐ für ein Wasserstoffatom, eine C₁-C₆-Alkylgruppe oder für eine Hydroxy-C₁-C₆-Alkylgruppe steht,
R_{b,} R_{c} unabhängig voneinander für für ein Wasserstoffatom, eine C₁-C₆-Alkylgruppe, eine Hydroxygruppe, ein Halogenatom aus der Gruppe Chlor, Brom, Fluor oder Jod oder für eine C₁-C₆-Alkoxygruppe stehen.

2. Verfahren nach Anspruch 1, **dadurch gekennzeichnet, dass** die erste Zusammensetzung (A) - bezogen auf das Gesamtgewicht der Zusammensetzung (A) - 0,01 bis 9,5 Gew.-%, bevorzugt 0,01 bis 8,0 Gew.-%, weiter bevorzugt 0,01 bis 6,0 und ganz besonders bevorzugt 0,01 bis 4,0 Gew.-% Wasser (A1) enthält.

3. Verfahren nach einem der Ansprüche 1 bis 2, **dadurch gekennzeichnet, dass** die erste Zusammensetzung (A) - bezogen auf das Gesamtgewicht der Zusammensetzung (A) - die organischen C₁-C₆-Alkoxysilane (A2) und/oder die Kondensationsprodukte hiervon in einer Gesamtmenge von 30,0 bis 85,0 Gew.-%, bevorzugt von 35,0 bis 80,0 Gew.-%, weiter bevorzugt von 40,0 bis 75,0 Gew.-%, noch weiter bevorzugt von 45,0 bis 70,0 Gew.-% und ganz besonders bevorzugt von 50,0 bis 65,0 Gew.-% enthält.

4. Verfahren nach einem der Ansrpüche 1 bis 3, **dadurch gekennzeichnet**, das die erste Zusammsensetzung (A) mindestens einen kosmetischen Inhaltsstoff aus der Gruppe aus Hexamethyldisiloxan. Octamethyltrisiloxan, Decamethyltetrasiloxan, Hexamethylcyclotrisiloxan, Octamethylcyclotetrasiloxan und Decamethylcyclopentasiloxan enthält.

5. Verfahren nach einem der Ansprüche 1 bis 4, **dadurch gekennzeichnet, dass** die erste Zusammensetzung (A) - bezogen auf das Gesamtgewicht der Zusammensetzung (A) - 10,0 bis 50,0 Gew.-%, bevorzugt 15,0 bis 45,0 Gew.-%, weiter bevorzugt 20,0 bis 40,0 Gew.-%, noch weiter bevorzugt 25,0 bis 35,0 Gew.-% und ganz besonders bevorzugt 31,0 bis 34,0 Gew.-% Hexamethyldisiloxan enthält.

6. Verfahren nach einem der Ansprüche 1 bis 5, **dadurch gekennzeichnet, dass** die zweite Zuammensetzung (B) - bezogen auf das Gesamtgewicht der Zusammsentzung (B) - 0,1 bis 95,0 Gew.-%, bevorzugt 20,0 bis 95,0 Gew.-%, weiter bevorzugt 30,0 bis 95,0 Gew.-% , noch weiter bevorzugt 50,0 bis 95,0 Gew.-% und ganz besonders bevorzugt 70,0 bis 95,0 Gew.- % Wasser (B1) enthält.

7. Verfahren nach einem der Ansprüche 1 bis 6, **dadurch gekennzeichnet, dass** die zweite Zusammensetzung (B) eine oder mehrere aromatische Verbindungen (B2) der Formel (AR-I) enthält, wobei
x für die Zahl 0 oder 1 steht.

8. Verfahren nach einem der Ansprüche 1 bis 7, **dadurch gekennzeichnet, dass** die zweite Zusammensetzung (B) eine oder mehrere aromatische Verbindungen (B2) der Formel (AR-I) enthält, wobei
x für die Zahl 0 steht und y für die Zahl 1 steht oder
x für die Zahl 1 steht und y für die Zahl 0 steht oder
x für die Zahl 0 steht und y für die Zahl 0 steht.

9. Verfahren nach einem der Ansprüche 1 bis 8, **dadurch gekennzeichnet, dass** die zweite Zusammensetzung (B) eine oder mehrere aromatische Verbindungen (B2) der Formel (AR-I) enthält, wobei
Rₐ für ein Wasserstoffatom oder für eine eine C₁-C₆-Alkylgruppe, besonders bevorzugt für ein Wasserstoffatom, für eine n-Pentylgruppe, für eine n-Butylgruppe, für eine n-Propylgruppe, für eine Ethylgruppe oder für eine Methylgruppe steht.

10. Verfahren nach einem der Ansprüche 1 bis 9, **dadurch gekennzeichnet, dass** die zweite Zusammensetzung (B) eine oder mehrere aromatische Verbindungen (B2) der Formel (AR-I) enthält, wobei
Rb, Rc unabhängig voneinander für ein Wasserstoffatom, für eine Hydroxygruppe oder für eine C₁-C₆-Alkylgruppe, ganz besonders bevorzugt für ein Wasserstoffatom oder für eine C₁-C₆-Alkylgruppe stehen.

11. Verfahren nach einem der Ansprüche 1 bis 10, **dadurch gekennzeichnet, dass** die zweite Zusammensetzung (B) eine oder mehrere aromatische Verbindungen (B2) der Formel (AR-I) enthält, die ausgewählt sind aus der Liste aus Thymol (2-Isopropyl-5-methyl-phenol), Benzylalkohol, Benzoesäure-n-pentylester, 4-Hydroxybenzoesäure- methylester, 4-Hydroxybenzoesäure- ethylester, 4-Hydroxybenzoesäure-n-propylester, Benzoesäure, Benzoesäuremethylester, Benzoesäure-ethylester, Benzoesäure-n-propylester, Benzoesäure-isopropylester, Benzoesäure-n-butylester, Benzoesäure-n-hexylester, 2-Hydroxybenzoesäure, 2-Hydroxybenzoesäuremethylester, 2-Hydroxybenzoesäure- ethylester, 2-Hydroxybenzoesäure- n-propylester, 2-Hydroxybenzoesäure-isopropylester, 2-Hydroxybenzoesäure-n-butylester, 2-Hydroxybenzoesäure-n-pentylester, 2-Hydroxybenzoesäure-n-hexylester, 3-Hydroxybenzoesäure, 3-Hydroxybenzoesäure- methylester, 3-Hydroxybenzoesäure- ethylester, 3-Hydroxybenzoesäure- n-propylester, 3-Hydroxybenzoesäure-isopropylester, 3-Hydroxybenzoesäure-n-butylester, 3-Hydroxybenzoesäure-n-pentylester, 3-Hydroxybenzoesäure-n-hexylester, 4-Hydroxybenzoesäure, 4-Hydroxybenzoesäure-isopropylester, 4-Hydroxybenzoesäure-n-butylester, 4-Hydroxybenzoesäure-n-pentylester, 4-Hydroxybenzoesäure-n-hexylester, 2-Methoxybenzoesäure, 2-Methoxybenzoesäure- methylester, 2-Methoxybenzoesäure- ethylester, 2-Methoxybenzoesäure- n-propylester, 2-Methoxybenzoesäure-isopropylester, 2-Methoxybenzoesäure-n-butylester, 2-Methoxybenzoesäure-n-pentylester, 2-Methoxybenzoesäure-n-hexylester, 3-Methoxybenzoesäure, 3-Methoxybenzoesäuremethylester, 3-Methoxybenzoesäure- ethylester, 3-Methoxybenzoesäure- n-propylester, 3-Methoxybenzoesäure-isopropylester, 3-Methoxybenzoesäure-n-butylester, 3-Methoxybenzoesäure-n-pentylester, 3-Methoxybenzoesäure-n-hexylester, 4-Methoxybenzoesäure, 4-Methoxybenzoesäure- methylester, 4-Methoxybenzoesäure-ethylester, 4-Methoxybenzoesäure- n-propylester, 4-Methoxybenzoesäure-isopropylester, 4-Methoxybenzoesäure-n-butylester, 4-Methoxybenzoesäure-n-pentylester und/oder 4-Methoxybenzoesäure-n-hexylester.

12. Verfahren nach einem der Ansprüche 1 bis 11, **dadurch gekennzeichnet, dass** die zweite Zusammensetzung (B) - bezogen auf das Gesamtgewicht der Zusammensetzung (B) - ein oder mehrere Aromatische Verbindungen (B2) der Formel (AR-I) in einer Gesamtmenge von 0,1 bis 35,0 Gew.-%, bevorzugt von 0,3 bis 15,0 Gew.-%, weiter bevorzugt von 0,5 bis 7,5 Gew.-% und ganz besonders bevorzugt von 1,0 bis 5,0 Gew.-% enthält.

13. Verfahren nach einem der Ansprüche 1 bis 12, bei dem auf dem keratinischen Material angewendet wird
- eine dritte Zusammensetzung (C), die enthält
mindestens eine farbgebende Verbindung as der Gruppe der Pigmente und/oder der direktziehenden Farbstoffe.

14. Verfahren nach Anspruch 13, **dadurch gekennzeichnet, dass** auf dem keratinischen Material eine Zusammensetzung angewendet wird, die unmittelbar vor der Anwendung durch Vermischen der ersten Zusammensetzung (A) mit der zweiten Zusammensetzung (B) und einer dritten Zusammensetzung (C) erhalten wurde.

15. Verfahren nach Anspruch 13, **dadurch gekennzeichnet, dass** in einem ersten Schritt auf dem keratinischen Material eine Zusammensetzung angewendet wird, die unmittelbar vor der Anwendung durch Vermischen der ersten Zusammensetzung (A) und der zweiten Zusammensetzung (B) hergestellt wurde, und in einem zweiten Schritt auf dem keratinischen Material die dritte Zusammensetzung (C) angewendet wird.

16. Verfahren nach einem der Ansprüche 1 bis 15, bei dem auf dem keratinischen Material angewendet wird
- eine vierte Zusammensetzung (D), die enthält
mindestens eine filmbildendes Polymer.

17. Verfahren nach einem der Ansprüche 1 bis 16, **dadurch gekennzeichnet, dass** die Zusammensetzung (B) und/oder die Zusammensetzung (C) mindestens eine farbgebende Verbindung aus der Gruppe der anorganischen Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

18. Verfahren nach einem der Ansprüche 1 bis 17, **dadurch gekennzeichnet, dass** die Zusammensetzung (B) und/oder die Zusammensetzung (C) mindestens eine farbgebende Verbindung aus der Gruppe der organischen Pigmente enthält, die ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

19. Verfahren nach einem der Ansprüche 1 bis 18, **dadurch gekennzeichnet, dass** die Zusammensetzung (B) und/oder die Zusammensetzung (C) mindestens eine farbgebende Verbindung aus der Gruppe der anionischen, nichtionischen, und/oder kationischen direktziehenden Farbstoffe enthält.

## Claims

1. A method for treating keratinous material, in particular human hair, wherein a composition is applied to the keratinous material, which composition was prepared immediately prior to application by mixing a first composition (A) and a second composition (B), wherein
- the first composition (A) contains, based on the total weight of composition (A)
(A1) less than 10% by weight of water and
(A2) at least one organic C₁-C₆ alkoxy silane selected from the group consisting of
- (3-aminopropyl)triethoxysilane
- (3-aminopropyl)trimethoxysilane
- (2-aminoethyl)triethoxysilane
- (2-aminoethyl)trimethoxysilane
- (3-dimethylaminopropyl)triethoxysilane
- (3-dimethylaminopropyl)trimethoxysilane
- (2-dimethylaminoethyl)triethoxysilane,
- (2-dimethylaminoethyl)trimethoxysilane
and/or their condensation products, and
at least one organic C₁-C₆-alkoxysilane selected from the group consisting of
- methyltrimethoxysilane
- methyltriethoxysilane
- ethyltrimethoxysilane
- ethyltriethoxysilane
- hexyltrimethoxysilane
- hexyltriethoxysilane
- octyltrimethoxysilane
- octyltriethoxysilane
- dodecyltrimethoxysilane,
- dodecyltriethoxysilane,
and/or their condensation products, and
- the second composition (B) contains
(B1) water and
(B2) one or more aromatic compounds of the formula (AR-I) 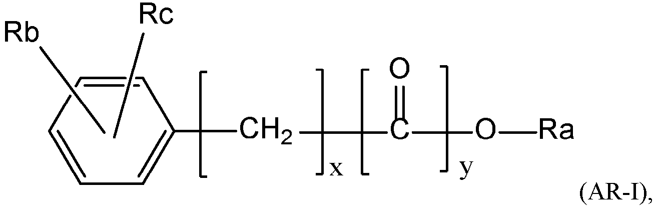
where
x is an integer from 0 to 3,
y is the number 0 or 1,
Rₐ is a hydrogen atom, a C₁-C₆ alkyl group or a hydroxy-C₁-C₆ alkyl group,
R_{b,} R_{c} independently of one another represent a hydrogen atom, a C₁-C₆ alkyl group, a hydroxy group, a halogen atom from the group consisting of chlorine, bromine, fluorine or iodine, or a C₁-C₆ alkoxy group.

2. Process according to claim 1, **characterised in that** the first composition (A) - based on the total weight of the composition (A) - contains 0.01 to 9.5 wt.%, preferably 0.01 to 8.0 wt.%, more preferably 0.01 to 6.0 wt.% and most preferably 0.01 to 4.0 wt.% water (A1).

3. Method according to one of claims 1 to 2, **characterised in that** the first composition (A) - based on the total weight of the composition (A) - contains the organic C₁-C₆ alkoxysilanes (A2) and/or the condensation products thereof in a total amount of 30.0 to 85.0 wt.%, preferably from 35.0 to 80.0 wt.%, more preferably from 40.0 to 75.0 wt.%, even more preferably from 45.0 to 70.0 wt.% and most preferably from 50.0 to 65.0 wt.%.

4. Method according to one of claims 1 to 3, **characterised in that** the first composition (A) contains at least one cosmetic ingredient from the group consisting of hexamethyldisiloxane, octamethyltrisiloxane, decamethyltetrasiloxane, hexamethylcyclotrisiloxane, octamethylcyclotetrasiloxane and decamethylcyclopentasiloxane.

5. Process according to one of claims 1 to 4, **characterised in that** the first composition (A) contains - based on the total weight of composition (A) - contains 10.0 to 50.0 wt.%, preferably 15.0 to 45.0 wt.%, more preferably 20.0 to 40.0 wt.%, even more preferably 25.0 to 35.0 wt.% and most preferably 31.0 to 34.0 wt.% hexamethyldisiloxane.

6. Method according to one of claims 1 to 5, **characterised in that** the second composition (B) - based on the total weight of composition (B) - contains
0.1 to 95.0 wt.%, preferably 20.0 to 95.0 wt.%, more preferably 30.0 to 95.0 wt.%, even more preferably 50.0 to 95.0 wt.% and most preferably 70.0 to 95.0 wt.% water (B1).

7. Method according to one of claims 1 to 6, **characterised in that** the second composition (B) contains one or more aromatic compounds (B2) of the formula (AR-I), wherein
x stands for the number 0 or 1.

8. Process according to one of claims 1 to 7, **characterised in that** the second composition (B) contains one or more aromatic compounds (B2) of the formula (AR-I), wherein
x is the number 0 and y is the number 1, or
x is the number 1 and y is the number 0, or
x represents the number 0 and y represents the number 0.

9. Method according to one of claims 1 to 8, **characterised in that** the second composition (B) contains one or more aromatic compounds (B2) of the formula (AR-I), wherein
Rₐ represents a hydrogen atom or a C₁-C₆ alkyl group, more preferably a hydrogen atom, an n-pentyl group, an n-butyl group, an n-propyl group, an ethyl group or a methyl group.

10. Method according to one of claims 1 to 9, **characterised in that** the second composition (B) contains one or more aromatic compounds (B2) of formula (AR-I), wherein
Rb, Rc independently represent a hydrogen atom, a hydroxy group or a C₁-C₆ alkyl group, more preferably a hydrogen atom or a C₁-C₆ alkyl group.

11. Process according to one of claims 1 to 10, **characterised in that** the second composition (B) contains one or more aromatic compounds (B2) of formula (AR-I) selected from the list comprising thymol (2-isopropyl-5-methylphenol), benzyl alcohol, benzoic acid n-pentyl ester, 4-hydroxybenzoic acid methyl ester, 4-hydroxybenzoic acid ethyl ester, 4-hydroxybenzoic acid n-propyl ester, benzoic acid, benzoic acid methyl ester, benzoic acid ethyl ester, benzoic acid n-propyl ester, benzoic acid isopropyl ester, benzoic acid n-butyl ester, benzoic acid n-hexyl ester, 2-hydroxybenzoic acid, 2-hydroxybenzoic acid methyl ester, 2-hydroxybenzoic acid ethyl ester, 2-hydroxybenzoic acid n-propyl ester, 2-hydroxybenzoic acid isopropyl ester, 2-hydroxybenzoic acid n-butyl ester, 2-hydroxybenzoic acid n-pentyl ester, 2-hydroxybenzoic acid n-hexyl ester, 3-hydroxybenzoic acid, 3-hydroxybenzoic acid methyl ester, 3-hydroxybenzoic acid ethyl ester, 3-hydroxybenzoic acid n-propyl ester, 3-hydroxybenzoic acid isopropyl ester, 3-hydroxybenzoic acid n-butyl ester, 3-hydroxybenzoic acid n-pentyl ester, 3-hydroxybenzoic acid n-hexyl ester, 4-hydroxybenzoic acid, 4-hydroxybenzoic acid isopropyl ester, 4-hydroxybenzoic acid n-butyl ester, 4-hydroxybenzoic acid n-pentyl ester, 4-hydroxybenzoic acid n-hexyl ester, 2-methoxybenzoic acid, 2-methoxybenzoic acid methyl ester, 2-methoxybenzoic acid ethyl ester, 2-methoxybenzoic acid n-propyl ester, 2-methoxybenzoic acid isopropyl ester, 2-methoxybenzoic acid n-butyl ester, 2-methoxybenzoic acid n-pentyl ester, 2-methoxybenzoic acid n-hexyl ester, 3-methoxybenzoic acid, 3-methoxybenzoic acid methyl ester, 3-methoxybenzoic acid ethyl ester, 3-methoxybenzoic acid n-propyl ester, 3-methoxybenzoic acid isopropyl ester, 3-methoxybenzoic acid n-butyl ester, 3-methoxybenzoic acid n-pentyl ester, 3-methoxybenzoic acid n-hexyl ester, 4-methoxybenzoic acid, 4-methoxybenzoic acid methyl ester, 4-methoxybenzoic acid ethyl ester, 4-methoxybenzoic acid n-propyl ester, 4-methoxybenzoic acid isopropyl ester, 4-methoxybenzoic acid n-butyl ester, 4-methoxybenzoic acid n-pentyl ester and/or 4-methoxybenzoic acid n-hexyl ester.

12. Method according to one of claims 1 to 11, **characterised in that** the second composition (B) - based on the total weight of composition (B) - contains one or more aromatic compounds (B2) of formula (AR-I) in a total amount of 0.1 to 35.0 wt.%, preferably from 0.3 to 15.0% by weight, more preferably from 0.5 to 7.5% by weight and most preferably from 1.0 to 5.0% by weight.

13. Method according to one of claims 1 to 12, in which
- a third composition (C) is applied to the keratinous material, which contains
at least one colouring compound from the group of pigments and/or direct dyes.

14. Process according to claim 13, **characterised in that** a composition obtained immediately prior to application by mixing the first composition (A) with the second composition (B) and a third composition (C) is applied to the keratinous material.

15. Method according to claim 13, **characterised in that**, in a first step, a composition is applied to the keratinous material, which was prepared immediately before application by mixing the first composition (A) and the second composition (B), and in a second step, the third composition (C) is applied to the keratinous material.

16. Process according to one of claims 1 to 15, in which the following is applied to the keratinous material
- a fourth composition (D) containing
at least one film-forming polymer.

17. Method according to one of claims 1 to 16, **characterised in that** composition (B) and/or composition (C) contains at least one colouring compound from the group of inorganic pigments selected from the group of coloured metal oxides, metal hydroxides, metal oxide hydrates, silicates, metal sulphides, complex metal cyanides, metal sulphates, bronze pigments and/or coloured pigments based on mica or glimmer, which are coated with at least one metal oxide and/or one metal oxychloride.

18. Method according to one of claims 1 to 17, **characterised in that** composition (B) and/or composition (C) contains at least one colouring compound from the group of organic pigments selected from the group consisting of carmine, quinacridone, phthalocyanine, sorghum, blue pigments with the Colour Index numbers CI 42090, CI 69800, CI 69825, CI 73000, CI 74100, CI 74160, yellow pigments with the Colour Index numbers CI 11680, CI 11710, CI 15985, CI 19140, CI 20040, CI 21100, CI 21108, CI 47000, CI 47005, green pigments with the Colour Index numbers CI 61565, CI 61570, CI 74260, orange pigments with the Colour Index numbers CI 11725, CI 15510, CI 45370, CI 71105, red pigments with the Colour Index numbers CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 and/or CI 75470.

19. Method according to one of claims 1 to 18, **characterised in that** composition (B) and/or composition (C) contains at least one colouring compound from the group of anionic, non-ionic and/or cationic direct dyes.

## Revendications

1. Procédé de traitement d'une matière kératinique, en particulier des cheveux humains, dans lequel on applique sur la matière kératinique une composition qui a été préparée immédiatement avant l'application en mélangeant une première composition (A) et une deuxième composition (B), dans lequel
- la première composition (A) contient, par rapport au poids total de la composition (A)
(A1) moins de 10 % en poids d'eau et
(A2) au moins un alcoxy silane organique en C₁ à C₆ choisi dans le groupe constitué par
- le (3-aminopropyl)triéthoxysilane
- le (3-aminopropyl)triméthoxysilane
- (2-aminoéthyl)triéthoxysilane
- (2-aminoéthyl)triméthoxysilane
- (3-diméthylaminopropyl)triéthoxysilane
- (3-diméthylaminopropyl)triméthoxysilane
- (2-diméthylaminoéthyl)triéthoxysilane,
- (2-diméthylaminoéthyl)triméthoxysilane
et/ou leurs produits de condensation, et
au moins un alcoxysilane organique en C₁ à C₆ choisi dans le groupe constitué par
- le méthyltriméthoxysilane
- méthyltriéthoxysilane
- éthyltriméthoxysilane
- éthyltriéthoxysilane
- hexyltriméthoxysilane
- hexyltriéthoxysilane
- octyltriméthoxysilane
- octyltriéthoxysilane
- dodécyltriméthoxysilane,
- dodécyltriéthoxysilane,
et/ou leurs produits de condensation, et
- la deuxième composition (B) contient
(B1) de l'eau et
(B2) un ou plusieurs composés aromatiques de formule (AR-I) 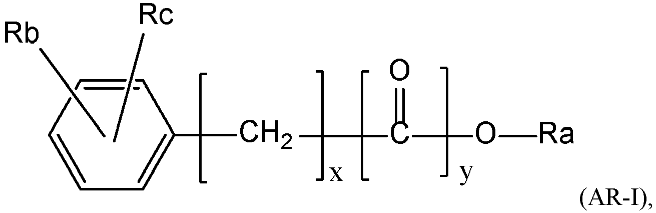
où
x représente un nombre entier de 0 à 3,
y représente le nombre 0 ou 1,
Rₐ représente un atome d'hydrogène, un groupe alkyle en C₁-C₆ ou un groupe hydroxyalkyle en C₁-C₆,
R_{b}, R_{c} représentent indépendamment l'un de l'autre un atome d'hydrogène, un groupe alkyle en C₁-C₆, un groupe hydroxy, un atome d'halogène choisi parmi le chlore, le brome, le fluor ou l'iode, ou un groupe alcoxy en C₁-C₆.

2. Procédé selon la revendication 1, **caractérisé en ce que** la première composition (A) - par rapport au poids total de la composition (A) - contient 0,01 à 9,5 % en poids, de préférence 0,01 à 8,0 % en poids, de manière encore plus préférée 0,01 à 6,0 % en poids et de manière particulièrement préférée 0,01 à 4,0 % en poids d'eau (A1).

3. Procédé selon l'une des revendications 1 à 2, **caractérisé en ce que** la première composition (A) - par rapport au poids total de la composition (A) - contient les alcoxysilanes organiques en C₁ à C₆ (A2) et/ou leurs produits de condensation en une quantité totale de 30,0 à 85,0 % en poids, de préférence de 35,0 à 80,0 % en poids, de préférence encore de 40,0 à 75,0 % en poids, de préférence encore plus de 45,0 à 70,0 % en poids et de préférence particulièrement de 50,0 à 65,0 % en poids.

4. Procédé selon l'une des revendications 1 à 3, **caractérisé en ce que** la première composition (A) contient au moins un ingrédient cosmétique choisi dans le groupe constitué par l'hexaméthyldisiloxane, l'octaméthyltrisiloxane, décaméthyltétrasiloxane, hexaméthylcyclotrisiloxane, octaméthylcyclotétrasiloxane et décaméthylcyclopentasiloxane.

5. Procédé selon l'une des revendications 1 à 4, **caractérisé en ce que** la première composition (A) contient, par rapport au poids total de la composition (A) - 10,0 à 50,0 % en poids, de préférence 15,0 à 45,0 % en poids, de préférence encore 20,0 à 40,0 % en poids, de préférence encore plus 25,0 à 35,0 % en poids et de manière particulièrement préférée 31,0 à 34,0 % en poids d'hexaméthyldisiloxane.

6. Procédé selon l'une des revendications 1 à 5, **caractérisé en ce que** la deuxième composition (B) contient, par rapport au poids total de la composition (B),
0,1 à 95,0 % en poids, de préférence 20,0 à 95,0 % en poids, de manière encore plus préférée 30,0 à 95,0 % en poids, de manière encore plus préférée 50,0 à 95,0 % en poids et de manière particulièrement préférée 70,0 à 95,0 % en poids d'eau (B1).

7. Procédé selon l'une des revendications 1 à 6, **caractérisé en ce que** la deuxième composition (B) contient un ou plusieurs composés aromatiques (B2) de formule (AR-I), dans laquelle
x représente le nombre 0 ou 1.

8. Procédé selon l'une des revendications 1 à 7, **caractérisé en ce que** la deuxième composition (B) contient un ou plusieurs composés aromatiques (B2) de formule (AR-I), dans laquelle
x représente le nombre 0 et y représente le nombre 1, ou
x représente le nombre 1 et y représente le nombre 0, ou
x représente le chiffre 0 et y représente le chiffre 0.

9. Procédé selon l'une des revendications 1 à 8, **caractérisé en ce que** la deuxième composition (B) contient un ou plusieurs composés aromatiques (B2) de formule (AR-I), où
Rₐ représente un atome d'hydrogène ou un groupe alkyle en C₁ à C₆, de préférence un atome d'hydrogène, un groupe n-pentyle, un groupe n-butyle, un groupe n-propyle, un groupe éthyle ou un groupe méthyle.

10. Procédé selon l'une des revendications 1 à 9, **caractérisé en ce que** la deuxième composition (B) contient un ou plusieurs composés aromatiques (B2) de formule (AR-I), dans laquelle
Rb, Rc représentent indépendamment l'un de l'autre un atome d'hydrogène, un groupe hydroxy ou un groupe alkyle en C₁-C₆, de préférence un atome d'hydrogène ou un groupe alkyle en C₁-C₆.

11. Procédé selon l'une des revendications 1 à 10, **caractérisé en ce que** la deuxième composition (B) contient un ou plusieurs composés aromatiques (B2) de formule (AR-I) choisis dans la liste comprenant le thymol (2-isopropyl-5-méthyl-phénol), alcool benzylique, ester n-pentyle de l'acide benzoïque, ester méthyle de l'acide 4-hydroxybenzoïque, ester éthyle de l'acide 4-hydroxybenzoïque, ester n-propyle de l'acide 4-hydroxybenzoïque, acide benzoïque, ester méthylique de l'acide benzoïque, ester éthylique de l'acide benzoïque, ester n-propylique de l'acide benzoïque, ester isopropylique de l'acide benzoïque, ester n-butylique de l'acide benzoïque, ester n-hexylique de l'acide benzoïque, acide 2-hydroxybenzoïque, ester méthylique de l'acide 2-hydroxybenzoïque, ester éthylique de l'acide 2-hydroxybenzoïque, ester n-propylique de l'acide 2-hydroxybenzoïque, ester isopropylique de l'acide 2-hydroxybenzoïque, ester n-butylique de l'acide 2-hydroxybenzoïque, ester n-pentyle de l'acide 2-hydroxybenzoïque, ester n-hexyle de l'acide 2-hydroxybenzoïque, acide 3-hydroxybenzoïque, ester méthylique de l'acide 3-hydroxybenzoïque, ester éthylique de l'acide 3-hydroxybenzoïque, ester n-propylique de l'acide 3-hydroxybenzoïque, ester isopropylique de l'acide 3-hydroxybenzoïque, ester n-butylique de l'acide 3-hydroxybenzoïque, ester n-pentylique de l'acide 3-hydroxybenzoïque, ester n-hexyle de l'acide 3-hydroxybenzoïque, acide 4-hydroxybenzoïque, ester isopropylique de l'acide 4-hydroxybenzoïque, ester n-butylique de l'acide 4-hydroxybenzoïque, ester n-pentyle de l'acide 4-hydroxybenzoïque, ester n-hexylique de l'acide 4-hydroxybenzoïque, acide 2-méthoxybenzoïque, ester méthylique de l'acide 2-méthoxybenzoïque, ester éthylique de l'acide 2-méthoxybenzoïque, ester n-propylique de l'acide 2-méthoxybenzoïque, ester isopropylique de l'acide 2-méthoxybenzoïque, ester n-butylique de l'acide 2-méthoxybenzoïque, ester n-pentyle de l'acide 2-méthoxybenzoïque, ester n-hexyle de l'acide 2-méthoxybenzoïque, acide 3-méthoxybenzoïque, ester méthylique de l'acide 3-méthoxybenzoïque, ester éthylique de l'acide 3-méthoxybenzoïque, ester n-propylique de l'acide 3-méthoxybenzoïque, ester isopropylique de l'acide 3-méthoxybenzoïque, ester n-butylique de l'acide 3-méthoxybenzoïque, ester n-pentylique de l'acide 3-méthoxybenzoïque, ester n-hexyle de l'acide 3-méthoxybenzoïque, acide 4-méthoxybenzoïque, ester méthylique de l'acide 4-méthoxybenzoïque, ester éthylique de l'acide 4-méthoxybenzoïque, ester n-propylique de l'acide 4-méthoxybenzoïque, ester isopropylique de l'acide 4-méthoxybenzoïque, ester n-butylique de l'acide 4-méthoxybenzoïque, ester n-pentylique de l'acide 4-méthoxybenzoïque et/ou ester n-hexyle de l'acide 4-méthoxybenzoïque.

12. Procédé selon l'une des revendications 1 à 11, **caractérisé en ce que** la deuxième composition (B) contient, par rapport au poids total de la composition (B), un ou plusieurs composés aromatiques (B2) de formule (AR-I) en une quantité totale de 0,1 à 35,0 % en poids, de préférence de 0,3 à 15,0 % en poids, de préférence encore de 0,5 à 7,5 % en poids et de manière particulièrement préférée de 1,0 à 5,0 % en poids.

13. Procédé selon l'une des revendications 1 à 12, dans lequel on applique sur le matériau kératinique
- une troisième composition (C) qui contient
au moins un composé colorant du groupe des pigments et/ou des colorants directs.

14. Procédé selon la revendication 13, **caractérisé en ce qu'**on applique sur le matériau kératinique une composition obtenue immédiatement avant l'application en mélangeant la première composition (A) avec la deuxième composition (B) et une troisième composition (C).

15. Procédé selon la revendication 13, **caractérisé en ce que**, dans une première étape, on applique sur la matière kératinique une composition obtenue immédiatement avant l'application par mélange de la première composition (A) et de la deuxième composition (B), et dans une deuxième étape, on applique sur la matière kératinique la troisième composition (C).

16. Procédé selon l'une des revendications 1 à 15, dans lequel on applique sur le matériau kératinique
- une quatrième composition (D) qui contient
au moins un polymère filmogène.

17. Procédé selon l'une des revendications 1 à 16, **caractérisé en ce que** la composition (B) et/ou la composition (C) contient au moins un composé colorant du groupe des pigments inorganiques, qui est choisi dans le groupe des oxydes métalliques colorés, des hydroxydes métalliques, des hydrates d'oxydes métalliques, des silicates, sulfures métalliques, cyanures métalliques complexes, sulfates métalliques, pigments de bronze et/ou de pigments colorés à base de mica ou de micacé, qui sont enrobés d'au moins un oxyde métallique et/ou un oxychlorure métallique.

18. Procédé selon l'une des revendications 1 à 17, **caractérisé en ce que** la composition (B) et/ou la composition (C) contient au moins un composé colorant du groupe des pigments organiques, qui est choisi dans le groupe constitué par le carmin, la quinacridone, phtalocyanine, sorgho, pigments bleus ayant les numéros d'index de couleur CI 42090, CI 69800, CI 69825, CI 73000, CI 74100, CI 74160, des pigments jaunes ayant les numéros d'index de couleur CI 11680, CI 11710, CI 15985, CI 19140, CI 20040, CI 21100, CI 21108, CI 47000, CI 47005, pigments verts portant les numéros d'index de couleur CI 61565, CI 61570, CI 74260, pigments orange portant les numéros d'index de couleur CI 11725, CI 15510, CI 45370, CI 71105, pigments rouges avec les numéros d'index de couleur CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

19. Procédé selon l'une des revendications 1 à 18, **caractérisé en ce que** la composition (B) et/ou la composition (C) contient au moins un composé colorant du groupe des colorants anioniques, non ioniques et/ou cationiques à fixation directe.
